# EUROPÄISCHE PATENTANMELDUNG

(11) **EP 3 242 538 A1**
(43) Veröffentlichungstag der Anmeldung: **08.11.2017**
(21) Anmeldenummer: 16179698.2
(22) Anmeldetag: 27.08.2013
(51) Int. Cl.: H05K 5/00, B23K 26/24, C03B 23/207, H05K 5/06, A61B 10/04

(54) **VERFAHREN ZUR HERSTELLUNG EINES HERMETISCHEN GEHÄUSES FÜR EIN ELEKTRONISCHES GERÄT**

(30) Priorität: 28.08.2012 AT 9372012; 11.09.2012 US 201261743744 P; 13.06.2013 AT 503862013; 13.06.2013 US 201361834514 P
(62) Teilanmeldung aus: 13753621.5
(71) Anmelder: mb-microtec ag, 3172 Niederwangen bei Bern (CH)
(72) Erfinder: Blunier, Heinz, 6333 Hünenberg See (CH); Kind, Hannes, 3012 Bern (CH); Schneider, Sandro M.O.L., 8800 Thalwil (CH)
(74) Vertreter: Willy, Otto Martin

(57) **Zusammenfassung**

Es wird ein Verfahren zur Herstellung eines Gehäuses (100..125) mit zumindest einem, zumindest einen Teil eines Innenraumes des Gehäuses (100..125) umfassenden hermetisch abgeschlossenen Aufnahmeraum (12, 19, 20) für ein elektronisches Gerät (3) angegeben. Dabei wird ein mindestens eine Öffnung aufweisender Hohlkörper (2) aus Glas hergestellt/bereitgestellt, zumindest ein elektronisches Geräts (3) durch die zumindest eine Öffnung eingebracht und der Aufnahmeraum (12, 19, 20) durch Verschmelzen des Gehäuses (100..125) hermetisch verschlossen oder die zumindest einen Öffnung mittels Laserstrahlung verschlossen. Weiterhin wird eine Vorrichtung mit einem zumindest zum Teil hermetisch abgeschlossenen Gehäuse (100..125) aus Silizium angegeben, das insbesondere nach dem genannten Verfahren hergestellt ist.

## Beschreibung

Die Erfindung betrifft ein Verfahren zur Herstellung eines hermetischen Gehäuses für ein elektronisches Gerät sowie eine Vorrichtung mit einem zumindest zum Teil hermetisch abgeschlossenen Gehäuse.

Hermetische Gehäuse für elektronische Geräte sind grundsätzlich bekannt. Sie schützen die elektronischen Geräte vor äußeren Einflüssen und sind flüssigkeitsdicht und/oder gasdicht. Umgekehrt kann auch der Schutz der Umgebung ein zu berücksichtigendes Kriterium beim Einsatz hermetischer Gehäuse sein, wenn das elektronische Gerät eine Gefahr für diese Umgebung darstellt. Als Beispiel werden medizinische Implantate angeführt (z.B. Herzschrittmacher, Insulinpumpen und dergleichen), bei denen das elektronische Gerät vor dem Kontakt mit Körperflüssigkeiten geschützt werden muss, andererseits aber auch der Organismus vor unkontrollierter Abgabe gefährlicher Stoffe durch das Gerät geschützt werden muss. Insbesondere ist bei einer solchen Anwendung auch darauf zu achten, dass das Gehäuse selbst keine gefährlichen Stoffe abgibt. Herkömmliche Herstellungsverfahren für solche Anordnungen sind somit vergleichsweise aufwändig.

Zur Herstellung derartiger, hermetisch geschlossener Gehäuse, insbesondere aus transparenten Materialien wie zum Beispiel Glas, ist es bereits bekannt, zum Verschließen dieser Gehäuse Laserstrahlungen, beispielsweise auch Ultrakurzimpulse von solchen Laserstrahlen zu verwenden. Derartige Verfahren und Vorrichtungen sind unter anderem aus der JP 2005/66629 A sowie der WO 2008/035770 oder auch der EP 1 741 510 A1 bekannt. Nachteilig ist bei diesen Verfahren und Vorrichtungen, dass diese nur zum Verbinden von ebenflächigen Bauteilen eingesetzt werden können.

Eine Aufgabe der Erfindung ist es daher, ein verbessertes Verfahren zur Herstellung eines hermetischen Gehäuses für ein elektronisches Gerät anzugeben.

Die Aufgabe der Erfindung wird durch ein Verfahren zur Herstellung eines Gehäuses mit zumindest einem, zumindest einen Teil eines Innenraumes des Gehäuses umfassenden hermetisch abgeschlossenen Aufnahmeraum für ein elektronisches Gerät gelöst, umfassend die Schritte:
- Herstellen/Bereitstellen eines mindestens eine Öffnung aufweisenden Hohlkörpers aus Glas,
- Einbringen, Positionieren und/oder Fixieren zumindest eines elektronischen Geräts durch die zumindest eine Öffnung
- hermetisches Verschließen des Aufnahmeraums durch Verschmelzen des Gehäuses oder
- Verschließen und Verschweißen der zumindest einen Öffnung mittels Laserstrahlung.

Dadurch kann auf einfache Weise ein hermetisches Gehäuse für ein elektronisches Gerät hergestellt werden. Durch Verschmelzen der Öffnung(en) kann das Gehäuse vollständig aus demselben Material, nämlich aus Glas, bestehen. Dadurch ist einerseits ein guter Schutz des elektronischen Geräts, andererseits aber auch ein guter Schutz der Umgebung, in der das Gerät betrieben wird, gewährleistet. Glas ist resistent gegen die meisten chemischen Substanzen und gibt selbst keine Stoffe ab, das heißt es ist inert. Insbesondere gelten die genannten Eigenschaften für Borsilikatglas bzw. andere dieselben Eigenschaften aufweisenden Glassorten.

Weitere vorteilhafte Ausgestaltungen des Herstellungsverfahrens ergeben sich aus der nachfolgenden Beschreibung, insbesondere im Zusammenhang mit den Figuren.

Vorteilhaft ist es, wenn zwischen dem Gerät und dem Schmelz- bzw. Schweißbereich ein Wärmeisolator oder eine Wärmeschutzschicht angeordnet wird, beziehungsweise wenn zwischen dem elektronischen Gerät und zumindest einer der beiden Stirnwände eine Wärmeisolationsvorrichtung angeordnet ist. Auf diese Weise kann das elektronische Gerät beim Trennbeziehungsweise Schweißvorgang wirkungsvoll vor der dabei entstehenden Hitze geschützt werden.

Günstig ist es, wenn in ein rohrförmiges Gehäuse durch die zumindest eine Öffnung mehrere elektronische Geräte mit Abstand in Längsrichtung hintereinander eingebracht, positioniert und/oder fixiert werden, worauf das Gehäuse mittels eines Laserstrahlung durch Abgabe von Nano- und/oder Piko-Sekunden Energieimpulsen oder bei kontinuierlicher Energieeinbringung im Zwischenbereich zwischen den elektronischen Geräten erwärmt und durch auf die Innen- und Außenseite des Gehäuses im Bereich der Trennstelle in Längsrichtung des Gehäuses ausgeübte unterschiedlich große Kräfte beidseits des Zwischenbereiches das rohrförmige Gehäuse mit einer Stirnwand verschlossen wird. Insbesondere können während des Trenn- und/oder Verschließvorgangs des rohrförmigen Gehäuses auf die Innen- und Außenseite des Gehäuses im Bereich der Trennstelle unterschiedlich große Kräfte in Längsrichtung des Gehäuses ausgeübt werden. Im Speziellen können während des Trenn- und/oder Verschließvorgangs des rohrförmigen Gehäuses die Innen- und Außenseite des Gehäuses im Bereich der Trennstelle mit einer unterschiedlich großen Druckkraft beaufschlagt werden. Auf diese Weise können die Gehäuse besonders rationell hergestellt werden. Insbesondere kann das Gehäuse durch die unterschiedlich großen Kräfte flexibel gestaltet werden.

In diesem Zusammenhang ist es auch von Vorteil, wenn eine Stirnwand des Gehäuses durch Erhitzen des Endbereiches des Mittelteils mittels eines Lasers durch Abgabe von Nano- und/oder Piko-Sekunden Energieimpulsen erwärmt und durch auf die Innen- und Außenseite des Gehäuses im Bereich der Trennstelleausgeübte unterschiedlich große Kräfte in Längsrichtung des Gehäuses eine Stirnwand gebildet wird. Dadurch kann die Stirnwand gebildet werden, ohne dass dazu ein gesondertes Bauteil vonnöten wäre.

Vorteilhaft ist es auch, wenn in den Innenraum eine Zwischenwand eingesetzt und mit dem Gehäuse verschweißt wird und die zwei Aufnahmeräume hermetisch voneinander getrennt werden. Insbesondere ist es dabei von Vorteil, wenn in einem ersten hermetisch abgeschlossenen Aufnahmeraum ein elektronisches Gerät und/oder eine Energiequelle angeordnet ist/sind. Von Vorteil ist es dabei insbesondere weiterhin, wenn in einem dem ersten hermetisch abgeschlossenen Aufnahmeraum benachbarten Aufnahmeraum zumindest eines der nachfolgend genannten Elemente, wie elektronisches Gerät, Analysevorrichtung, Messwertaufnehmer, angeordnet wird. Dadurch ist es möglich, verschiedene Bestandteile des elektronischen Geräts in unterschiedlichen Aufnahmeräumen anzuordnen. Beispielsweise kann ein Gassensor in einem Aufnahmeraum und eine Auswerteelektronik in einem anderen Aufnahmeraum angeordnet werden, um die Auswerteelektronik beispielsweise vor reaktiven Gasen, die an den Gassensor gelangen, zu schützen.

Besonders vorteilhaft ist es, wenn das Gehäuse zumindest eine von Außen sich zumindest in einem der beiden Teilräume erstreckende Öffnung aufweist, die luft- und/oder flüssigkeitsdurchlässig ist. Auf diese Weise kann eine Verbindung von der Analysevorrichtung oder dem Messwertaufnehmer zur Umgebung des Gehäuses vorgesehen werden. Beispielsweise kann ein Gassensor so die das Gehäuse umgebende Luft untersuchen. Selbstverständlich ist die Messung nicht auf Gase beschränkt, sondern es können auch Flüssigkeiten untersucht werden. Beispielsweise kann der Sensor als ph-Wert-Sensor für Flüssigkeiten ausgebildet werden.

Vorteilhaft ist es in diesem Zusammenhang, wenn die Analyse- und Auswerteeinheit zur Analyse und Auswertung von Körperflüssigkeiten und/oder Gewebeproben ausgebildet ist. Aufgrund der geringen Abmessungen des Gehäuses kann dieses nämlich gut im menschlichen oder tierischen Körper eingesetzt werden.

Besonders vorteilhaft ist es, wenn in der Zwischenwand zwischen dem ersten hermetischen Aufnahmeraum und dem zweiten hermetischen Aufnahmeraum Öffnungen angeordnet sind, die durch Lichtleiter, elektrische Leitungen oder leitende Kontaktmassen hermetisch verschlossen werden. Dadurch können zum Beispiel Daten und/oder Energie zwischen den beiden Aufnahmeräumen ausgetauscht werden. Beispielsweise können elektrische, optische, pneumatische oder hydraulische Leitungen vorgesehen sein.

Vorteilhaft ist es, wenn der Taupunkt des Wasserdampfes in der Luft im Gehäuse zumindest in dessen hermetisch abgeschlossenen Teilraum bei 0 °C bevorzugt zwischen - 10°C und-30°C beträgt. Auf diese Weise kann ein Beschlagen der Innenwände des Gehäuses bei dessen Betriebstemperatur verhindert werden.

Vorteilhaft ist es weiterhin, wenn der Teilraum wasserdampfdicht mit einer Wasserdampfdurchlässigkeit s_{d} größer als 2.500m ausgebildet ist. Auf diese Weise können insbesondere ein Eindiffundieren von Wasserdampf in das Gehäuse und in Folge eine unerwünschte Erhöhung der Luftfeuchtigkeit innerhalb desselben vermieden werden.

Günstig ist es, wenn eine Kamera in den Hohlraum eingebracht wird beziehungsweise wenn im Gehäuse eine Bildaufnahmeeinrichtung zum Beispiel ein Bilderkennungschip angeordnet ist. Auf diese Weise kann die entstandene Vorrichtung zur Beobachtung eingesetzt werden, wobei sich das durchsichtige Gehäuse als besonderer Vorteil erweist.

Günstig ist es auch, wenn ein Funkmodul und/oder ein Transponder in den Hohlraum eingebracht wird/werden. Dadurch ist es möglich, dass Daten aus dem elektronischen Gerät ausgelesen oder in dieses übertragen werden.

Besonders vorteilhaft ist es, wenn eine motorisch bewegte Masse in den Hohlraum eingebracht wird. Auf diese Weise ist es möglich, das Gehäuse mit dem elektronischen Gerät fortzubewegen. Dazu kann die genannte Masse translatorisch und/oder rotatorisch bewegt werden. Durch die entstehende Gegenkraft beziehungsweise das entstehende Gegendrehmoment wir das Gehäuse mit dem elektronischen Gerät in Bewegung versetzt. Besonders vorteilhaft ist bei diesem Antrieb, dass das Gehäuse keinerlei Öffnungen nach außen aufweisen muss.

Vorteilhaft ist es auch, wenn ein Rückstoßantrieb mit einer Durchtrittsöffnung in dem Gehäuse in den Hohlraum eingebracht wird. Dies ist eine weitere Möglichkeit, um das Gehäuse mit dem elektronischen Gerät in Bewegung zu versetzen. Beispielweise können dazu Pumpen, Verdichter aber auch unter Druck stehende Behälter, deren Inhalt kontrolliert abgelassen wird, eingesetzt werden. Vorteilhaft kann mit einem solchen Rückstoßantrieb eine stetige Antriebskraft realisiert werden.

Besonders vorteilhaft ist es, wenn im Hohlkörper eine Tritiumgaslichtquelle angeordnet wird. Insbesondere wenn im Hohlkörper auch eine Kamera angeordnet ist, resultiert solcherart eine besonders vorteilhafte Anordnung, da die Tritiumgaslichtquelle das Sichtfeld der Kamera ausleuchten kann, ohne dass dazu externe Energie nötig wäre. Alternativ oder zusätzlich kann in dem Gehäuse auch eine LED oder ein Leuchtstoff mit Nachleuchteigenschaften angeordnet sein.

Günstig ist es dabei, wenn der Teilraum gasdicht, insbesondere Tritiumgasdicht, ausgebildet ist. Dadurch kann das Entweichen eines Gases, insbesondere des eine Zerfallsstrahlung abgebenden Tritium-Gases, in einen anderen Teilraum oder in die Umgebung des Gehäuses vermieden werden.

Günstig ist es weiterhin, wenn zumindest einer der nachstehend Bauteile im Gehäuse, wie Lichtquelle, Bilderkennungsvorrichtung, Bildaufnahmevorrichtung, elektronischen Gerät, Analyse-, Speicher- und Auswerteeinheit mit einem Sender zum drahtlosen Übermitteln von Daten verbunden ist. Auf diese Weise können ermittelte Daten an externe Auswerteeinheiten weitergeleitet werden.

Günstig ist es auch, wenn zumindest einer der nachstehend Bauteile im Gehäuse, wie Lichtquelle, Bilderkennungsvorrichtung, Bildaufnahmevorrichtung, elektronischen Gerät, Analyse- und Auswerteeinheit mit einer Energiequelle verbunden ist. Auf diese Weise können die im Gehäuse angeordneten Baugruppen ohne externe Energieversorgung betrieben werden.

Besonders vorteilhaft ist es, wenn die Energiequelle mit einem berührungslos beaufschlagbaren Energieumsetzer verbunden ist. Auf diese Weise kann Energie in das Innere des Gehäuses übertragen werden ohne die Gehäusewand dafür durchbrechen zu müssen. Beispielsweise kann die Energie induktiv ,zB. Infrarot oder Ultraschall, übertragen werden. Denkbar ist auch, dass der Energieumsetzer Bewegungsenergie des Gehäuses in elektrische Energie umsetzt, so wie dies beispielsweise von Automatikuhren bekannt ist.

Vorteilhaft ist es, wenn zur Herstellung des Gehäuses wenigstens ein Ende eines rohrförmigen Grundkörpers verschmolzen/verschweißt wird. Dadurch ist es möglich, ein Ende oder beide Enden des Rohres ohne die Hilfe von Zusatzstoffen zu verschließen.

Besonders vorteilhaft ist es, wenn zur Herstellung des Gehäuses ein rohrförmiger Grundkörper durch Aufschmelzen getrennt wird, wobei der geschmolzene Werkstoff die entstehenden Enden verschließt. Dadurch ist die Herstellung der Gehäuse besonders rationell möglich, da in einem Arbeitsschritt der rohrförmige Grundkörper und gleichzeitig je ein Ende von zwei Gehäusen geformt wird. Das Rohr selbst kann neben kreisförmigem Querschnitt beispielsweise auch einen polygonförmigen, insbesondere quadratischen, sowie ovalen Querschnitt aufweisen.

Besonders vorteilhaft ist es in diesem Zusammenhang, wenn der rohrförmige Grundkörper während des Trennvorgangs im Bereich der Trennstelle auf Zug beansprucht wird. Dadurch wird der Trennvorgang beziehungsweise auch das Verschließen der entstehenden Rohrenden begünstigt.

Günstig ist es, wenn eine Öffnung im Gehäuse mit Hilfe eines Lasers verschmolzen/verschweißt wird. Dadurch ist es möglich, die Öffnung ohne die Hilfe von Zusatzstoffen zu verschließen.

Vorteilhaft ist es, wenn zumindest eine der Stirnwände konvex ausgebildet ist. Insbesondere ist es von Vorteil, wenn die Stirnwände mit einem sich in die vom Mittelteil abgewendeten Richtung erstreckenden Konus oder als Kugelkalotte bzw. Kugelkalottenabschnitt ausgebildet sind. Dadurch wird eine große Druckstabilität und Resistenz gegenüber Implosion und Explosion erreicht. Günstig ist es aber auch, wenn zumindest eine der Stirnwände alternativ konkav oder ebenflächig ausgebildet ist.

Vorteilhaft ist es auch, wenn das Gehäuse aus einem zylindrischen Mitteilteil und zwei in den Stirnseiten desselben angeordneten Stirnwänden gebildet ist. Dadurch kann das Gehäuse aus einem rohrförmigen Grundkörper hergestellt werden.

Vorteilhaft ist es, wenn eine Wandstärke des Gehäuses zumindest im Mittelbereich zwischen 0,05 bis 5 mm beträgt. Dadurch kann eine hinreichende Stabilität gegenüber den häufigsten im Betrieb auftretenden Beanspruchungen erzielt werden.

Vorteilhaft ist es, wenn in dem Gehäuse Mikrobohrungen angeordnet werden, die gasdurchlässig jedoch flüssigkeitsundurchlässig sind. Auf diese Weise kann das elektronische Gerät gekühlt werden, insbesondere auch dann, wenn es in einer Flüssigkeit betrieben wird.

Besonders vorteilhaft ist es, wenn das Gehäuse mit dem elektronischen Gerät in ein weiteres hermetisches Glasgehäuse eingebracht wird, in dem Mikrobohrungen angeordnet werden, die gasdurchlässig jedoch flüssigkeitsundurchlässig sind. Auf diese Weise kann das elektronische Gerät gekühlt werden, ohne dass dieses dem durch die Mikrobohrungen hindurch tretenden Gas ausgesetzt wird. Dies ist insbesondere bei empfindlichen elektronischen Geräten und/oder aggressiven Gasen von Vorteil.

Günstig ist es, wenn in dem Gehäuse Bohrungen für den Durchtritt metallischer Drähte und/oder Lichtleitfasern angeordnet werden. Auf diese Weise können elektrische Energie beziehungsweise Strahlungsenergie und/oder Daten durch das Gehäuse hindurch übertragen werden.

Vorteilhaft ist es, wenn die äußere Oberfläche des Gehäuses mit einem Gel und/oder einem Geschmacksträger beschichtet wird. Das in das Glasgehäuse gekapselte elektronische Gerät kann für den Einsatz im menschlichen oder tierischen Körper, insbesondere für das Verschlucken durch den Mensch oder das Tier, vorgesehen sein. Um dieses Verschlucken zu begünstigen beziehungsweise zu erleichtern, kann die Oberfläche des Gehäuses wie erwähnt mit einem Gel und/oder einem Geschmacksträger beschichtet sein.

Vorteilhaft ist es auch, wenn die äußere Oberfläche des Gehäuses aufgeraut und/oder mit reaktiven, ein Anwachsen in menschlichen/tierischen/pflanzlichen Gewebe begünstigen Substanzen/Strukturen versehen wird. Diese Variante ist insbesondere dann von Vorteil, wenn das im Glasgehäuse gekapselte elektronische Gerät ortsfest im Gewebe verankert werden soll, insbesondere durch einen operativ-invasiven Eingriff.

Günstig ist es, wenn das Gehäuse aus zwei Gehäuseteilen besteht und die beiden Gehäuseteile mittels Laserstrahlung mit Energieabgabe im Nano- und/oder Piko-Sekundenbereich, gepulst oder kontinuierlich, miteinander verschweißt werden. Auf diese Weise kann die zum Verschweißen nötige Energie gut dosiert und das elektronische Gerät somit effektiv von Hitzeeinwirkung geschützt werden. Von Vorteil ist es dabei, wenn die Abgabe der Energie der Laserimpulse mit einer Steuervorrichtung derart gesteuert wird, dass die Temperatur im Inneren des Gehäuses in einem Bereich der gleich oder größer 2 mm von der Schweißnaht oder der Einwirkstelle der Laserstrahlen entfernt ist unter einem Wert von 200° gehalten wird. In diesem Fall kann mit sehr hoher Wahrscheinlichkeit davon ausgegangen werden, dass das elektronische Gerät durch die Einwirkung der Laserstrahlung keinen Schaden nimmt.

Besonders vorteilhaft ist es, wenn während des Aufbringens der Wärmeenergie durch die Laserstrahlung die der Schweißnaht benachbarten Bereiche gekühlt werden. Auf diese Weise kann das elektronische Gerät noch besser vor der durch die Laserstrahlung entstehenden Hitze geschützt werden.

Vorteilhaft ist es, wenn das Verfahren zur Herstellung eines hermetischen Gehäuses für ein elektronisches Gerät folgende Schritte umfasst:
- Anfertigen zumindest einer Vertiefung in zumindest einem Gehäuseteil eines Gehäuses,
- Herstellen zumindest eines Hohlraums durch Zusammenfügen der Gehäuseteile, wobei zumindest eine Öffnung, insbesondere zumindest zwei Öffnungen, von aussen in den Hohlraum offen bleibt/bleiben,
- Einbringen eines elektronischen Gerätes in den zumindest einen Hohlraum durch die zumindest eine Öffnung und
- Verschließen und Verschweißen der zumindest einen Öffnung mittels Laserstrahlung.
Auf diese Weise können insbesondere quaderförmige beziehungsweise planare Gehäuse für elektronische Geräte geformt werden. Dabei ist es insbesondere von Vorteil, wenn der eine Gehäuseteil durch eine plattenförmige Deckschicht, gebildet wird.

Vorteilhaft ist es, wenn das genannten Verfahren zusätzlich folgende Schritte umfasst:
- Herstellen einer fluoreszierenden und/oder phosphoreszierenden aus einem durch Zerfallsstrahlung zum Leuchten anregbaren Stoff gebildeten Schicht auf wenigstens einer Begrenzungswand des zumindest einen Hohlraums und
- Einbringen eines eine Zerfallsstrahlung für einen zum Leuchten anregbaren Stoff abgebendes Medium in den zumindest einen Hohlraum durch die zumindest eine Zuleitungsöffnung.
Auf diese Weise kann das Gehäuse mit selbstleuchtenden Flächen ausgestattet werden. Durch selektives Beschichten wenigstens eines Gehäuseteils mit einem fluoreszierenden und/oder phosphoreszierenden Stoff kann die lichtabgebende Fläche darüber hinaus gut strukturiert werden. Dadurch können unter anderem beliebige leuchtende Buchstaben, Ziffern, Symbole oder sonstige geometrische Flächen realisiert werden.

Vorteilhaft ist auch ein Verfahren zur Herstellung selbstleuchtender Körper, umfassend die Schritte:
- Anfertigen zumindest einer Vertiefung in zumindest einem Gehäuseteil eines Gehäuses,
- Herstellen einer fluoreszierenden und/oder phosphoreszierenden aus einem durch Zerfallsstrahlung zum Leuchten anregbaren Stoff gebildeten Schicht und/oder einer Maske auf wenigstens einem Teil einer Begrenzungswand zumindest eines Hohlraums,

- Herstellen des zumindest eines Hohlraums durch Zusammenfügen der Gehäuseteile
- Verschweißen der Gehäuseteile mittels einer Laserstrahlung durch Abgabe von Nano- und/oder Piko-Sekunden Energieimpulsen und/oder kontinuierlich, wobei zumindest eine Zuleitungsöffnung, insbesondere zumindest zwei Zuleitungsöffnungen, von aussen in den Hohlraum offen bleibt/bleiben,
- Einbringen eines eine Zerfallsstrahlung für einen zum Leuchten anregbaren Stoff abgebendes Medium oder des Stoffs und des Mediums in den zumindest einen Hohlraum durch die zumindest eine Zuleitungsöffnung und
- Verschließen und Verschweißen der zumindest einen Zuleitungsöffnung mittels Hitzeeinwirkung durch Laserstrahlung und/oder eine Gasflamme.

Günstig ist es dabei, wenn die Laserstrahlung durch Nano- und/oder Piko-Sekunden Energieimpulsen und/oder kontinuierlich gebildet wird, da die zugeführte Laserleistung auf diese Weise gut beeinflusst werden kann.

Günstig ist es, wenn zumindest ein Teil des Gehäuses bzw. der Deckschicht diffus hergestellt wird. Dadurch kann die flächige Lichtabstrahlung noch weiter verbessert werden.

Günstig ist es aber auch, wenn zumindest ein Teil des Gehäuses bzw. der Deckschicht opak ausgebildet ist. Auf diese Weise kann das Durchdringen von Licht durch Bereiche des Gehäuses oder auch durch das ganze Gehäuse verhindert werden. Insbesondere eignen sich diese opaken Bereiche zur Bildung einer Maske.

Günstig ist es, wenn die diffusen oder opaken Teile des Gehäuses bzw. der Deckschicht den Schmelz- bzw. Schweißbereichen benachbart angeordnet sind. Dadurch ist es möglich, diffuse und opake Gehäuseteile miteinander zu verbinden und so verschiedene Teilbereiche des Gehäuses zu schaffen.

Vorteilhaft ist es auch, wenn zumindest ein Teil des Gehäuses bzw. der Deckschicht mit einer Funktionsbeschichtung, z.B. einer Folie, versehen ist. Insbesondere kann die Funktionsbeschichtung, z.B. die Folie, diffus oder opak ausgebildet sein. Auf diese Weise ist es beispielsweise möglich, ein Gehäuse mit an sich im Wesentlichen homogenen optischen Eigenschaften optisch unterschiedlich zu strukturieren. Dazu wird das Gehäuse beispielsweise einfach beschichtet und/oder mit einer Folie beklebt. Selbstverständlich ist die Funktionsbeschichtung und/oder Folie nicht auf optische Eigenschaften beschränkt, sondern kann auch andere physikalische Eigenschaften beeinflussen, zum Beispiel die elektrische Leitfähigkeit.

Günstig ist es, wenn das zumindest zum Teil hermetisch abgeschlossene Gehäuse aus Silizium einteilig aus einem einzigen Grundwerkstoff hergestellt ist. Da das Gehäuse einteilig ist, kann die Dichtheit desselben besonders gut gewährleistet werden.

Günstig ist es weiterhin, wenn das zumindest zum Teil hermetisch abgeschlossenen Gehäuse aus Silizium aus mehreren Gehäuseteilen besteht und zumindest aus einem einzigen Grundwerkstoff hergestellt ist. Dadurch kann die Herstellung des Gehäuses auf einfache Weise erfolgen, da das Fügen der Teile wegen Verwendung nur eines Werkstoffs in der Regel unproblematisch ist.

Günstig ist es auch, wenn die Gehäuseteile aus verschiedenen Werkstoffen gebildet sind, die im Wesentlichen dieselben physikalischen und chemischen Eigenschaften aufweisen. Dadurch kann die Herstellung des Gehäuses ebenfalls auf einfache Weise erfolgen, da das Fügen der Teile aus Werkstoffen mit ähnlichen Eigenschaften in der Regel unproblematisch ist.

Vorteilhaft ist es, wenn die fluoreszierende und/oder phosphoreszierende Schicht durch Beschichtung des Gehäuseteils und/oder der Gehäuseteile mit einem Klebstoff und anschließendem Aufbringen eines fluoreszierenden und/oder phosphoreszierenden Stoffs auf die Klebeschicht hergestellt wird. Dadurch können auch fluoreszierende und/oder phosphoreszierende Medien für die Beschichtung verwendet werden, welche keine oder nur geringe Haft- oder Klebeeigenschaften aufweisen. Die Beschichtung des Gehäuseteils mit dem fluoreszierenden und/oder phosphoreszierenden Stoff (z.B. ZnS und/oder ZnO) kann beispielsweise durch Stempeln erfolgen sowie durch Sputtern.

Besonders vorteilhaft ist es, wenn der Klebstoff und/oder der fluoreszierende und/oder phosphoreszierende Stoff beziehungsweise die fluoreszierende/phosphoreszierende Schicht vor der Herstellung des Hohlraums auf das Gehäuseteil und/oder die Gehäuseteile aufgebracht wird. Dadurch kann das Gehäuseteil und/oder die Gehäuseteile auf vergleichsweise einfache Weise selektiv mit Klebstoff und/oder einem fluoreszierenden und/oder phosphoreszierenden Stoff beschichtet werden. Beispielsweise können die Schichten aufgesprüht oder aufgewalzt werden, insbesondere unter Zuhilfenahme von Masken. Denkbar ist es beispielsweise auch, die Schichten aufzudrucken oder aufzustempeln.

Günstig ist es auch, wenn der Klebstoff und/oder der fluoreszierende und/oder phosphoreszierende Stoff beziehungsweise die fluoreszierende und/oder phosphoreszierende Schicht durch die zumindest eine Zuleitungsöffnung in den (fertigen) Hohlraum eingebracht wird. Dadurch können großflächige (und insbesondere unstrukturierte) Leuchtschichten auf einfache Weise hergestellt werden.

Besonders günstig ist es, wenn der Klebstoff vor der Herstellung des Hohlraums auf das Gehäuseteil und/oder die Gehäuseteile aufgebracht wird und der fluoreszierende und/oder phosphoreszierende Stoff durch die zumindest eine Zuleitungsöffnung in den Hohlraum eingebracht wird. Bei dieser Variante wird also die selektive Benetzung des Gehäuseteils und/oder der Gehäuseteile mit Klebstoff mit einer einfachen Abscheidung des fluoreszierenden und/oder phosphoreszierenden Stoffs kombiniert. Solcherart resultiert ein einfaches Verfahren zur Herstellung strukturierter leuchtender Flächen.

Generell kann der fluoreszierende und/oder phosphoreszierende Stoff als Pulver, als Gel, in Gasphase oder als Lösung eingebracht werden. Insbesondere kann dabei das Fließverhalten des genannten Stoffes durch Vorsehen einer Mikro- oder Nanostruktur verbessert werden.

Vorteilhafte Möglichkeiten zum Aufbringen des fluoreszierenden und/oder phosphoreszierenden Stoffs auf den Gehäuseteil sind Aufsputtern, Aufdampfen, Aufsprühen, Walzen oder Spin-Coaten.

Günstig ist es, wenn die Vertiefung in das Gehäuseteil mechanisch (zB. durch fräsen, ultraschallbohren) oder mit Hilfe eines Ionenstrahls-, Laserstrahlabtrag, Powder-Blasting oder chemisches Ätzen hergestellt wird. Alle Verfahren ermöglichen das Herstellen einer Vertiefung im Rahmen eines bewährten und damit kontrolliert ablaufenden Herstellungsprozesses.

Vorteilhaft ist es, wenn zwischen den Gehäuseteilen oder auf denselben eine Maske angeordnet wird. Insbesondere kann die Maske auch zwischen der fluoreszierenden und/oder phosphoreszierenden Schicht und zumindest einem Gehäuseteil angeordnet sein. Damit ist es möglich, beispielsweise leuchtende Buchstaben, Ziffern, Symbole und geometrische Flächen herzustellen, ohne dass dazu die fluoreszierende und/oder phosphoreszierende Schicht strukturiert werden müsste. Stattdessen wird eine unstrukturierte fluoreszierende und/oder phosphoreszierende Schicht mit einer strukturierten Maske, welche das erzeugte Licht partiell durchlässt oder teilweise reflektiert oder absorbiert, kombiniert.

Günstig ist es, wenn die Gehäuseteile sowie gegebenenfalls die Maske mittels Fusion Bonding, z.B. bei Temperaturen von 700-800°C, miteinander verbunden werden. Dabei werden die Grenzflächen der verbundenen Teile durch die Van der Waal'sche Kräfte zusammengehalten.

Günstig ist es auch, wenn die Gehäuseteile und die sowie gegebenenfalls die Maske mittels Anodischem Bonding z.B. bei Temperaturen von 350-450°C miteinander verbunden werden. Bei diesem Verfahren wird eine chemische Bindung an den Grenzflächen der zu verbindenden Teile durch elektrische Anziehungskräfte, das heißt durch Anlegen einer elektrischen Spannung, eingeleitet.

Vorteilhaft ist es, wenn die zumindest eine Zuleitungsöffnung mit Hilfe eines Lasers verschweißt wird. Dadurch ist es möglich, die Zuleitungsöffnung ohne die Hilfe von Zusatzstoffen zu verschließen. An dieser Stelle wird angemerkt, dass Verfahren zum Verschweißen von Glasteilen mit Hilfe eines Lasers an sich bekannt sind, beispielsweise aus der EP 1 741 510 A1.

Günstig ist es, wenn
- für ein Gehäuseteil Glas oder Silizium vorgesehen wird und/oder
- für ein Gehäuseteil Glas oder Borsilikat vorgesehen wird und/oder
- als fluoreszierender und/oder phosphoreszierender Stoff Zinksulfid (ZnS) eingebracht wird und/oder

- als Klebstoff Phosphorsäure (H₃PO₄) aufgebracht wird und/oder
- als Zerfallstrahlung abgebendes Medium Tritium-Gas eingebracht wird.
Insbesondere durch die Verwendung von Zinksulfid (ZnS) und Tritium-Gas wird der selbstleuchtende Körper mit Mitteln realisiert, welche im Zusammenhang mit Tritium-Gas-Leuchten bewährt sind, sodass auch von einer hohen Zuverlässigkeit des vorgestellten Gehäuses ausgegangen werden kann.

Vorteilhaft ist es dabei, wenn als Klebstoff Phosphorsäure (H₃PO₄) aufgebracht wird und als fluoreszierender und/oder phosphoreszierender Stoff Zinksulfid (ZnS) eingebracht wird. Günstig wirkt sich dabei aus, dass Phosphorsäure an sich keine übermäßigen Hafteigenschaften aufweist und erst in Kombination mit Zinksulfid (ZnS) eine haftende Schicht ausbildet. Der in Form von Phosphorsäure vorliegende Klebstoff kann somit sehr differenziert aufgetragen werden, wodurch feine Strukturen hergestellt werden können. Beispielsweise kann die Phosphorsäure mit Hilfe des Tintenstrahldruck-Verfahrens aufgetragen werden.

Vorteilhaft ist es aber auch wenn als fluoreszierende und/oder phosphoreszierende Schicht ein Gemisch aus Phosphorsäure (H₃PO₄) und Zinksulfid (ZnS) aufgebracht wird. Bei dieser Variante wird also eine von Haus aus klebende Substanz auf ein Gehäuseteil aufgebracht. Diese Variante eignet sich daher insbesondere zum Aufdrucken (z.B. mit Hilfe des Kalanderdruck-Verfahrens oder Aufstempeln).

Günstig ist es, wenn zumindest einer der Gehäuseteile mit über die Fläche des Hohlraumes verteilt angeordneten Stützelementen versehen ist, die sich in Richtung des anderen Gehäuseteiles erstrecken und die Gehäuseteile über diese Stützelemente aufeinander abgestützt werden. Auf diese Weise wird vermieden, dass die Gehäuseteile übermäßig gegeneinander durchbiegen können.

Günstig ist es dabei weiterhin, wenn zumindest einer der beiden Gehäuseteile mit den Stützelementen verbunden ist. Dadurch können die Stützelemente gut im Hohlraum positioniert werden. Sind die Stützelemente mit beiden Gehäuseteilen verbunden, können zwischen diesen auch Zug- und Scherkräfte verbessert übertragen werden.

Günstig ist es wenn das Gehäuse als Quader oder Scheibe ausgebildet ist, insbesondere wenn der Quader oder die Scheibe durch zwei im Wesentlichen plattenförmige Gehäuseteile mit einer mehreckigen oder elliptischen oder kreisrunden Grundfläche gebildet wird und die Summe der zur Grundfläche senkrechten Höhen der beiden Gehäuseteile geringer ist als eine kürzere Seitenlänge bzw. ein minimaler Durchmesser oder Radius derselben. Auf diese Weise ist die lichtabgebende Fläche im Verhältnis zum Volumen des selbstleuchtenden Körpers relativ groß.

Ergänzend wird angemerkt, dass sich die zum Verfahren offenbarten Ausführungsvarianten und die daraus resultierenden Vorteile gleichermaßen auf die zum selbstleuchtenden Körper präsentierten Varianten sowie Vorteile beziehen und umgekehrt.

Zum besseren Verständnis der Erfindung wird diese anhand der nachfolgenden Figuren näher erläutert.

Es zeigen jeweils in stark schematisch vereinfachter Darstellung:
- Fig. 1: einen rohrförmigen Grundkörper mit mehreren darin angeordneten elektronischen Geräten;
- Fig. 2: die Anordnung aus Fig. 1, welche in einem Bereich zwischen zwei elektronischen Geräten von einem Laser bestrahlt wird;
- Fig. 3: die Anordnung aus Fig. 2 mit einer beginnenden Einschnürung im Trennbereich;
- Fig. 4: die Anordnung aus Fig. 2 mit einer fortgeschrittenen Einschnürung im Trennbereich;
- Fig. 5: die Anordnung aus Fig. 2 mit vollständig durchtrenntem Grundkörper;
- Fig. 6: die Anordnung aus Fig. 5 mit einem fertiggestellten Gehäuse;
- Fig. 7: wie Fig. 1, nur mit durchgehender Leiterplatte;
- Fig. 8: wie Fig. 7, nur mit aufgetrenntem Rohrkörper und durchtrennter Leiterplatte;
- Fig. 9: wie Fig. 1, nur mit Tritiumgaslichtquellen und thermischen Trennwänden;
- Fig. 10: eine Kühlmöglichkeit für den Rohrkörper;
- Fig. 11: ein beispielhaftes elektronisches Gerät im Detail;
- Fig. 12: ein Gehäuse mit einer konischen Stirnwand;
- Fig. 13: ein Gehäuse mit überlappenden Verbindungsbereich;
- Fig. 14: Antriebsmöglichkeiten für das elektronische Gerät mit Hilfe von bewegten Massen;
- Fig. 15: Antriebsmöglichkeiten für das elektronische Gerät nach dem Rückstoßprinzip;
- Fig. 16: Gehäuse mit Mikrobohrungen;
- Fig. 17: ein quaderförmiges Gehäuse für ein elektronisches Gerät in Explosionsdarstellung;
- Fig. 18: ein beispielhaftes elektronisches Gerät mit einem Gehäuse in Form eines Ziffernblatts;
- Fig. 19: wie Fig. 17, nur mit einer anders ausgebildeten Zuleitungsöffnung;
- Fig. 20: eine Variante eines planaren Gehäuses für ein elektronisches Gerät;
- Fig. 21: ein weiteres beispielhaftes elektronisches Gerät mit einem Gehäuse in Form eines Ziffernblatts;
- Fig. 22: ein planares zweiteiliges Gehäuse mit Mikrobohrungen bzw. Durchtrittsbohrungen;
- Fig. 23: ein Gehäuse, das aus zwei im Wesentlichen U-Profil-förmigen Teilen besteht;
- Fig. 24: ein Gehäuse, das einen Gehäuseteil ähnlich einer Eprouvette und einen damit verbundenen plattenförmigen Gehäuseteil umfasst und
- Fig. 25: ein Gehäuse, das einen rohrförmigen Gehäuseteil umfasst, der beidseits mit plattenförmigen Gehäuseteilen verschlossen ist.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten des Gehäuses für das elektronische Gerät, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

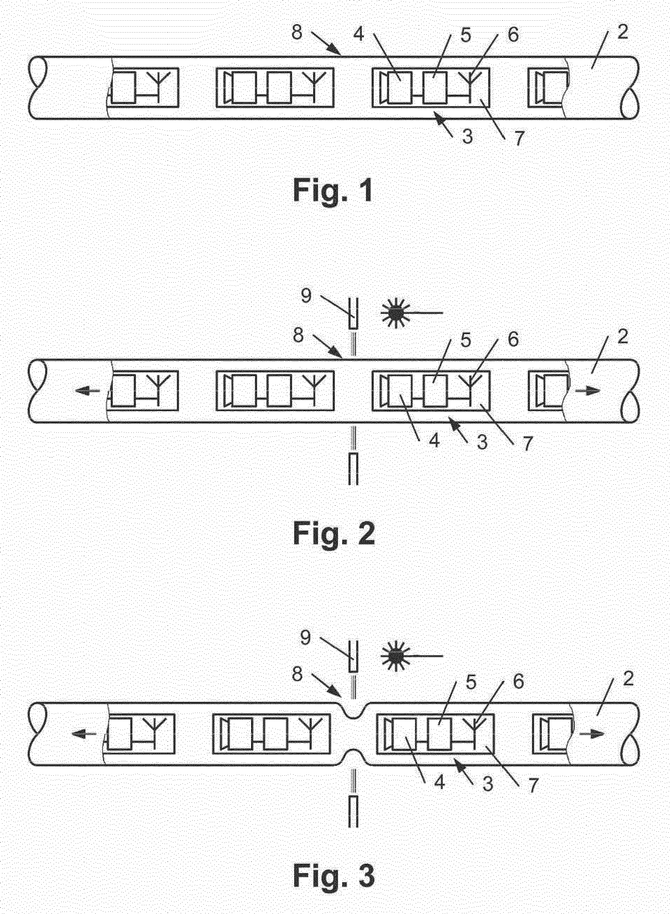

Die Figuren 1 bis 6 zeigen eine Variante zur Herstellung eines elektronischen Geräts mit einem Gehäuse 100 aus Glas. In einem ersten Schritt wird ein Hohlkörper 2 aus Glas hergestellt beziehungsweise bereitgestellt. In diesem Fall wird angenommen, dass es sich dabei um ein Rohr 2 aus Glas handelt. In einem weiteren Schritt wird zumindest ein elektronisches Gerät 3 durch eine Öffnung des Rohres 2 eingebracht. Wie aus Fig. 1 ersichtlich ist, werden gleich mehrere voneinander beabstandete Geräte 3 eingebracht.

Das beispielhafte elektronische Gerät 3 umfasst eine Kamera 4 (respektive eine Bildaufnahmeeinrichtung wie zum Beispiels einen Bilderkennungschip), einen Mikroprozessor 5 sowie eine Antenne 6. In diesem Beispiel wird angenommen, dass der Mikroprozessor 5 auch Komponenten zur Funkkommunikation umfasst. Gemeinsam mit der Antenne 6 wird solcherart ein Funkmodul und/oder ein Transponder gebildet. Die Kamera, 4 der Mikroprozessor 5 und die Antenne 6 sind auf je einer Platine 7 herkömmlicher Bauart angeordnet.

In Fig. 2 ist dargestellt, dass der Bereich zwischen zwei elektronischen Geräten 3 im Schmelzbereich 8 mit einem Laser 9 bestrahlt wird. In vorteilhafter Weise können auf das rohrförmige Gehäuse 2, z.B. ein Rohr mit zylindrischem oder mehreckigem Querschnitt, während des Trenn- und/oder Verschließvorgangs des rohrförmigen Gehäuses 2 auf die Innen- und Außenseite des Gehäuses 2 im Bereich der Trennstelle 8 unterschiedlich große Kräfte in Längsrichtung des Gehäuses 2 ausgeübt werden. So ist es beispielsweise möglich die zu trennenden Teile des rohrförmigen Gehäuses 2 mit einer Zugbeanspruchung (siehe Pfeile) oder die Innen- und Außenseite des Gehäuses 2 im Bereich der Trennstelle 8 mit einer unterschiedlich großen Druckkraft zu beaufschlagen.

In Fig. 3 ist dargestellt, dass die Bestrahlung mit dem Laser 9 Schmelzbereich 8 und die damit einhergehende Erwärmung beziehungsweise Plastifikation des Glases in Kombination mit der Zugbeanspruchung zu einer Verlängerung und Einschnürung des Rohres 2 führt.

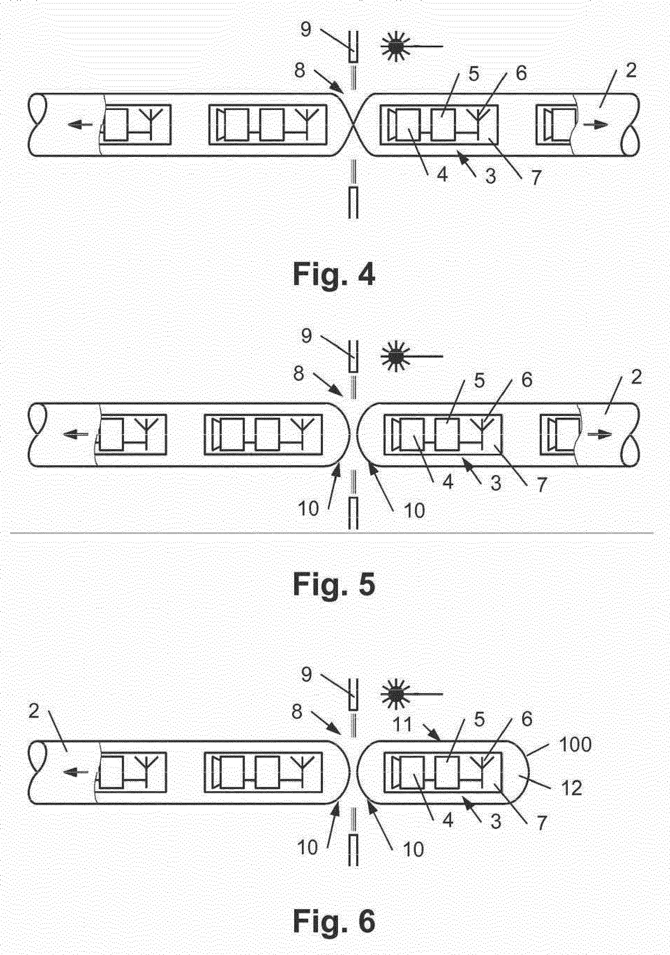

In Fig. 4 ist eine noch weitere Einschnürung des Rohres 2 und in Fig. 5 schließlich eine Trennung des Rohres 2 dargestellt. Wie aus der Fig. 5 ersichtlich ist, wird bei diesem Vorgang das Rohr 2 nicht nur getrennt, sondern es werden gleichzeitig auch die entstandenen Enden durch den erweichten/aufgeschmolzenen Werkstoff verschlossen, wodurch an der Trennstelle 8 Stirnwände 10 und das Gehäuse 100 entstehen.

Dieser Vorgang wird nun in jedem Bereich zwischen zwei elektronischen Geräten 3 wiederholt, sodass Zug um Zug vollständig geschlossene und einen Hohlraum 12 einschließende Glasgehäuse 100 um die elektronischen Geräte 3 gebildet werden, wie dies in Fig. 6 dargestellt ist. Das Gehäuse 100 besteht in diesem Fall aus einem zylindrischen Mitteilteil und zwei in den Stirnseiten desselben angeordneten konvexen Stirnwänden 10, welche im Wesentlichen die Form einer Kugelkalotte beziehungsweise eines Kugelkalottenabschnitts haben. Insbesondere können die Stirnwände 10 durch Abgabe von Energie mit Nano- oder Piko-Sekundenimpulsen und/oder bei kontinuierlicher Energieeinbringung z.B. mittels einer Gasflamme gebildet werden. Das Gehäuse 100 ist einteilig aus einem einzigen Grundwerkstoff hergestellt, in diesem Beispiel aus Glas. Es könnte beispielsweise aber auch aus Silizium hergestellt sein.

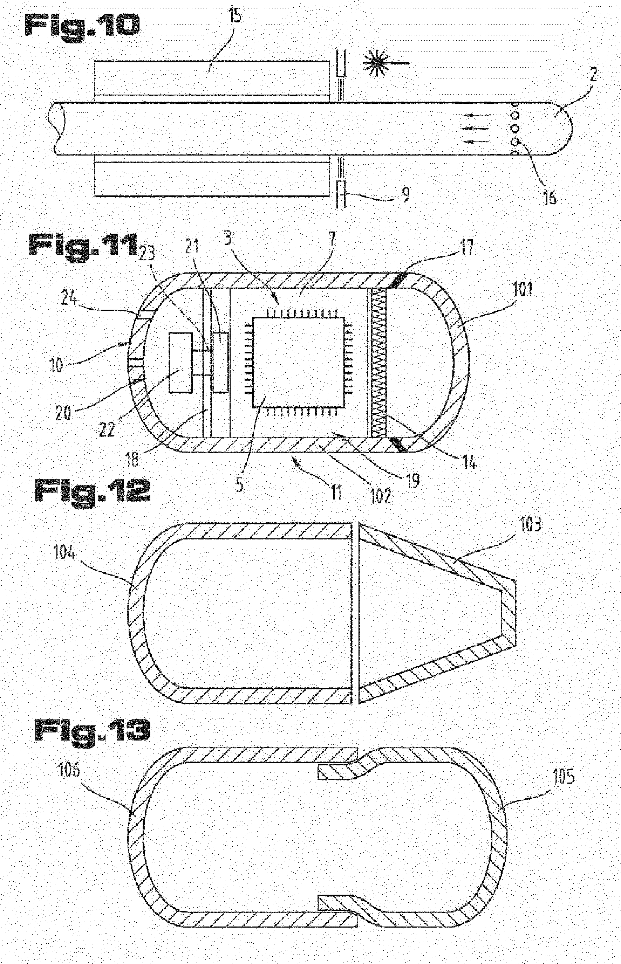

Denkbar ist natürlich auch, dass das Gehäuse 100 aus mehreren Bestandteilen aufgebaut ist. Beispielsweise können der Mittelteil 11 und die Stirnwände 10 als gesonderte Bauteile vorliegen. Weitere Beispiele für mehrteilige Gehäuse sind in den Figuren 10 bis 13 und 17 bis 23 dargestellt. Für eine gute Verbindung der Gehäuseteile ist es von Vorteil, wenn diese Aus einem einzigen Grundwerkstoff hergestellt sind (z.B. Glas oder Silizium) oder wenigstens im Wesentlichen dieselben physikalischen und chemischen Eigenschaften aufweisen.

In dem vorangegangenen Beispiel wurde der Hohlkörper 2 nur auf Zug beansprucht. Denkbar ist aber auch, dass der Hohlkörper 2 alternativ oder zusätzlich mit einem erhöhten Luftdruck beaufschlagt wird, sodass außerhalb des Hohlkörpers ein höherer Luftdruck herrscht als innerhalb. Auf diese Weise kann ebenfalls eine Einschnürung des Hohlkörpers 2 bei Erwärmung im Schmelzbereich 8 begünstigt werden.

Denkbar ist weiterhin, dass zwischen dem Hohlkörper 2 und dem Laser 9 eine Relativdrehung um die Achse des Hohlkörpers 2 vollführt wird. Es kann also ein Laser 9 oder es können mehrere Laser 9 um den Hohlkörper 2 herum gedreht werden, oder es wird der Hohlkörper 2 selbst gegenüber dem Laser 9 / den Lasern 9 gedreht. Auf diese Weise kann der Schmelzbereich 8 gleichmäßig erwärmt werden.

Das Verfahren zur Herstellung eines Gehäuses 100 mit zumindest einem, zumindest einen Teil eines Innenraumes des Gehäuses 100 umfassenden hermetisch abgeschlossenen Aufnahmeraum 12 für ein elektronisches Gerät 3, umfasst somit die Schritte:
- Herstellen/Bereitstellen eines mindestens eine Öffnung aufweisenden Hohlkörpers 2 aus Glas,
- Einbringen, Positionieren und/oder Fixieren zumindest eines elektronischen Geräts 3 durch die zumindest eine Öffnung
- hermetisches Verschließen des Aufnahmeraums 12 durch Verschmelzen des Gehäuses 100 oder
- Verschließen und Verschweißen der zumindest einen Öffnung mittels mittels Hitzeeinwirkung durch Laserstrahlung und/oder eine Gasflamme.

Vorteilhaft ist es dabei, wenn die Laserstrahlung in Nano- und/oder Piko-Sekunden-Energieimpulsen und/oder bei kontinuierlicher Energieeinbringung abgegeben wird, da die abgegebene Leistung dadurch gut beeinflusst werden kann.

Vorteilhaft ist es auch, wenn während des Trenn- und/oder Verschließvorgangs des rohrförmigen Gehäuses 100 auf die Innen- und Außenseite des Gehäuses 100 im Bereich der Trennstelle 8 unterschiedlich große Kräfte in Längsrichtung des Gehäuses 100 ausgeübt werden. Insbesondere können während des Trenn- und/oder Verschließvorgangs des rohrförmigen Gehäuses 100 die Innen- und Außenseite des Gehäuses 100 im Bereich der Trennstelle 8 mit einer unterschiedlich großen Druckkraft beaufschlagt werden. Somit kann eine Stirnwand 10 des Gehäuses 100 durch Erhitzen des Endbereiches des Mittelteils 11 mittels eines Lasers durch Abgabe von Nano- und/oder Piko-Sekunden Energieimpulsen und/oder bei kontinuierlicher Energieeinbringung erwärmt und durch auf die Innen- und Außenseite des Gehäuses 100 im Bereich der Trennstelle 10 ausgeübte unterschiedlich große Kräfte in Längsrichtung des Gehäuses 100 eine Stirnwand 10 gebildet werden. Insbesondere können also in ein rohrförmiges Gehäuse 100 durch die zumindest eine Öffnung mehrere elektronische Geräte 3 mit Abstand in Längsrichtung hintereinander eingebracht, positioniert und/oder fixiert werden, worauf das Gehäuse 100 mittels eines Laserstrahlung durch Abgabe von Nano- und/oder Piko-Sekunden Energieimpulsen im Zwischenbereich 8 zwischen den elektronischen Geräten 3 erwärmt und durch auf die Innen- und Außenseite des Gehäuses 100 im Bereich der Trennstelle 8 in Längsrichtung des Gehäuses 100 ausgeübte unterschiedlich große Kräfte beidseits des Zwischenbereiches das rohrförmige Gehäuse 100 mit einer Stirnwand 10 verschlossen wird.

Vorteilhaft ist es, wegen der Kamera 3, wenn der Taupunkt des Wasserdampfes in der Luft im Gehäuse zumindest in dessen hermetisch abgeschlossenen Teilraum 0 °C bevorzugt zwischen - 10°C und -30°C beträgt. Damit ist die Restfeuchte im Gehäuse 100 so gering, dass es zu keiner Kondensation oder zu keinem Beschlagen der Innenseite des Gehäuses 100 kommen kann.

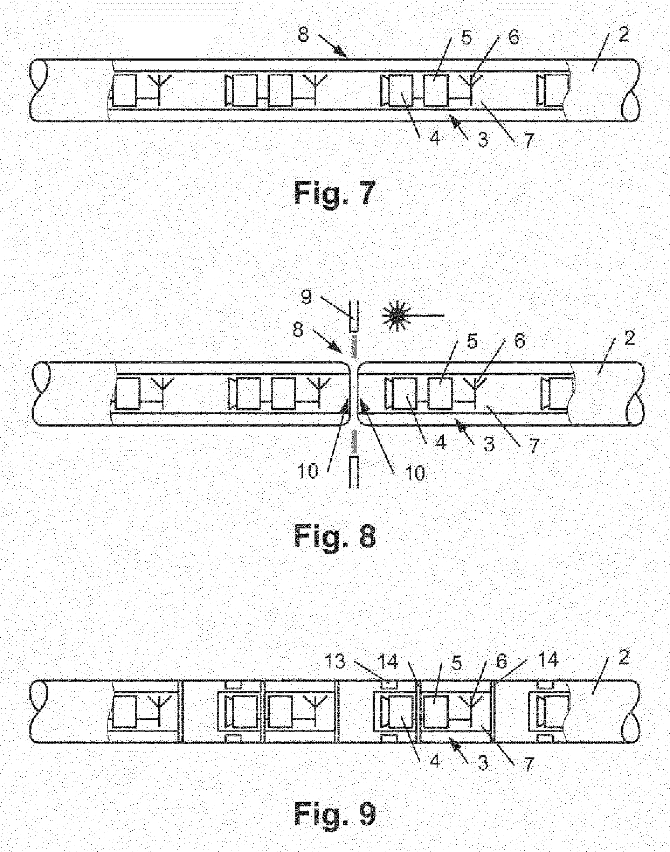

Die Figuren 7 und 8 zeigen nun ein ganz ähnliches Herstellungsverfahren wie die Figuren 1 bis 6, allerdings sind die elektronischen Geräte 3 nun auf einer gemeinsamen Platine 7 angeordnet, die durch den Laser 9 im Schmelzbereich 8 getrennt wird. Somit wird in einem Arbeitsschritt die Platine 7 getrennt und ein Gehäuse 100 für das elektronische Gerät 3 gebildet, wie dies in Fig. 8 dargestellt ist. Auch hier werden die Enden des Rohres 2 durch den erweichten/aufgeschmolzenen Werkstoff verschlossen und im Trennbereich 8 Stirnwände 10 gebildet. Die Stirnwände 10 sind dabei im Wesentlichen eben. Generell kann eine Stirnwand 10 natürlich nicht nur konvex, sondern auch konkav ausgebildet sein.

Generell ist anzumerken, dass der Trennvorgang in der Praxis nicht wie in den Figuren 1 bis 8 dargestellt in der Mitte des Rohres 2, sondern normalerweise an einem Ende desselben gestartet wird, sodass auf einer Seite des Lasers 9 stets ein Gehäuse fertiggestellt wird. In der Praxis erfolgt die Herstellung der Gehäuse 100 somit wie in Fig. 6 dargestellt, wobei das Rohr 2 jeweils um eine Position nach rechts verschoben wird. Das erste offenen Ende des Rohres 2 (beim Starten des Produktionsprozesses) wird dabei nicht notwendiger Weise durch ein Trennen des Rohres 2 verschlossen, sondern kann einfach verschmolzen/verschweißt werden.

Fig. 9 zeigt eine Erweiterung der elektronischen Geräte 3 dahingehend, dass im Bereich der Kamera 4 eine Tritiumgaslichtquelle 13 angeordnet ist und thermische Barrieren beziehungsweise Isolationen 14 vorgesehen sind. Letztere sollen empfindliche Geräte wie zum Beispiel den Mikroprozessor 5 vor übermäßiger Erwärmung durch den Laser 9 schützen. Selbstverständlich kann eine Isolation 14 auch vor der Kamera 4 angeordnet sein, wenn diese eine Öffnung für das Objektiv der Kamera 4 aufweist oder in diesem Bereich durchsichtig ist.

Zur Bildung einer Tritiumgaslichtquelle 13 kann das Rohr 2 im Bereich der Kamera 4 wie in Fig. 9 dargestellt beispielsweise ringförmig mit einer fluoreszierenden und/oder phosphoreszierenden Schicht aus einem durch Zerfallsstrahlung zum Leuchten anregbaren Stoff versehen werden und das Gehäuse 100 des elektronischen Geräts 3 mit einem eine Zerfallsstrahlung abgebenden Medium gefüllt werden. Beispielsweise können dazu Öffnungen im Rohr 2 vorgesehen sein, die nach dem Füllvorgang verschlossen werden (vergleiche auch Fig. 10 sowie Fig. 17 bis 23). Alternativ kann als Lichtquelle auch eine LED oder ein Leuchtstoff mit Nachleuchteigenschaften vorgesehen sein.

Vorteilhaft ist es, wenn als fluoreszierender und/oder phosphoreszierender Stoff Zinksulfid (ZnS), als Klebstoff für das ZnS Phosphorsäure (H₃PO₄) und als eine Zerfallsstrahlung abgebendes Medium Tritium-Gas verwendet wird. Selbstverständlich sich aber auch andere Kombinationen zur Herstellung eines selbstleuchtenden Bereichs prinzipiell anwendbar.

Fig. 10 zeigt eine Möglichkeit zur Vermeidung übermäßiger Erwärmung des Rohres 2 außerhalb des Schmelzbereichs 8. Zu diesem Zweck kann eine Kühlmanschette 15 vorgesehen sein. In diesem Beispiel ist diese nur links vom Schmelzbereichs 8 angeordnet, selbstverständlich kann diese aber auch beidseits des Schmelzbereichs 8 angeordnet sein.

Zusätzlich oder alternativ dazu, kann das Rohr 2 während des Trennvorgangs auch von einem Kühlmedium durchströmt werden. Dazu sind spezielle Spülbohrungen 16 vorgesehen. Als Spülmedium kommt beispielsweise Stickstoff (N₂), Argon (Ar) oder Helium (He) in Betracht. Die Spülbohrungen 16 können, wenn sie nicht mehr benötigt werden, verschweißt werden. Beispielsweise kann dies mit dem Laser 9, einem anderen Laser oder überhaupt auf andere Art und Weise erfolgen. Beispielsweise können die Spülbohrungen 16 auch verklebt sein.

Von Vorteil ist es auch, wenn die Abgabe der Energie der Laserimpulse mit einer Steuervorrichtung derart gesteuert wird, dass die Temperatur im Inneren des Gehäuses 100 in einem Bereich der gleich oder größer 2 mm von der Einwirkstelle der Laserstrahlen entfernt ist unter einem Wert von 200° gehalten wird, da das elektronische Gerät 3 dann keinen Schaden nimmt.

Ein Vorteil des präsentierten Verfahrens ist es auch, dass der Wasserdampfgehalt in dem hergestellten Gehäuse 100 durch Verwendung eines trockenen Spül- beziehungsweise Füllgases wie N₂ oder He sehr niedrig ist. Vorteilhaft kann damit eine Kondensation von Wasserdampf an der Innenseite des Gehäuses 100 bei der Betriebstemperatur des elektronischen Gerätes 3 vermieden werden.

Fig. 11 zeigt ein beispielhaftes und schematisch dargestelltes elektronisches Gerät 3 mit einem aus zwei Gehäuseteilen 101 und 102 aus Glas nun im Detail. Die beiden Gehäuseteile 101, 102 sind in diesem Beispiel mit Hilfe einer Schweißnaht 17 verbunden, welche insbesondere mit einem Laser hergestellt sein kann. Vorteilhaft ist die Schweißnaht 17 dazu etwas geneigt angeordnet.

Neben der thermischen Isolation 14 umfasst das Gehäuse 101, 102 auch eine mit diesem fix verbundene Trennwand 18, wodurch ein erster hermetischer Hohlraum 19 und ein zweiter Hohlraum 20 entstehen. Im ersten Hohlraum 19 befinden sich ein Mikroprozessor 5 und eine Energiequelle 21 eines elektronischen Geräts 3. Im zweiten Hohlraum 20 befindet sich ein Sensor 22, welcher über Leitungen 23 mit der Energiequelle 21 respektive dem Mikroprozessor 5 verbunden ist. Die Leitungen 23 sind dabei so durch die Trennwand 18 geführt, dass kein substantieller Austausch von Gasen zwischen dem Hohlraum 19 und dem Hohlraum 20 stattfinden kann. Über Durchtrittsbohrungen 24 steht der Sensor 22 in mehr oder minder direktem Kontakt mit der Umgebung des Gehäuses 101, 102. Die Öffnung 24 kann luft- und/oder flüssigkeitsdurchlässig sein. Der Sensor 22 kann somit beispielsweise als Gassensor, Drucksensor, ph-Wert-Sensor oder ähnliches ausgebildet sein.

Die Leiter 23 können beispielsweise zur Energieübertragung und/oder Datenübertragung genützt werden. Generell können die Leiter 23 als Drähte ausgebildet sein. Denkbar ist aber auch, dass eine leitfähige Paste oder ein leitfähiger Kleber in die entsprechenden Bohrungen eingebracht wird, welche/welcher einerseits die elektrische Verbindung zur Platine 7, andererseits gleichzeitig auch für die elektrische Verbindung durch die Trennwand 18 hindurch vorgesehen ist. Beispielsweise kann eine Lötpaste eingebracht und erwärmt (aufgeschmolzen) werden. Zusätzlich oder alternativ kann die Bohrung auch metallisch beschichtet werden. Generell können zur Herstellung einer leitfähigen Verbindung durch die Trennwand 18 Prozesse angewandt werden, wie sie auch beispielsweise aus der Leiterplattenfertigung zur Herstellung sogenannter "Vias" oder Durchkontaktierungen verwendet werden.

Prinzipiell können in der Trennwand 18 Bohrungen zum Durchtritt beliebiger Medien vorgesehen sein. Beispielseiweise können Leitungen für den Flüssigkeits- oder Gastransport vorgesehen sein. Denkbar ist aber natürlich auch, dass Lichtleitfasern durch die Bohrungen hindurchgeführt werden.

Die genannten Techniken eignen sich natürlich auch uneingeschränkt für den Durchtritt beliebiger Leitungen durch das Gehäuse 101, 102 hindurch.

Insbesondere, wenn Tritium-Gas-Lichtquellen 13 eingesetzt werden, ist es von Vorteil, wenn die Teilräume 19, 20 gasdicht, insbesondere Tritiumgasdicht, ausgebildet sind.

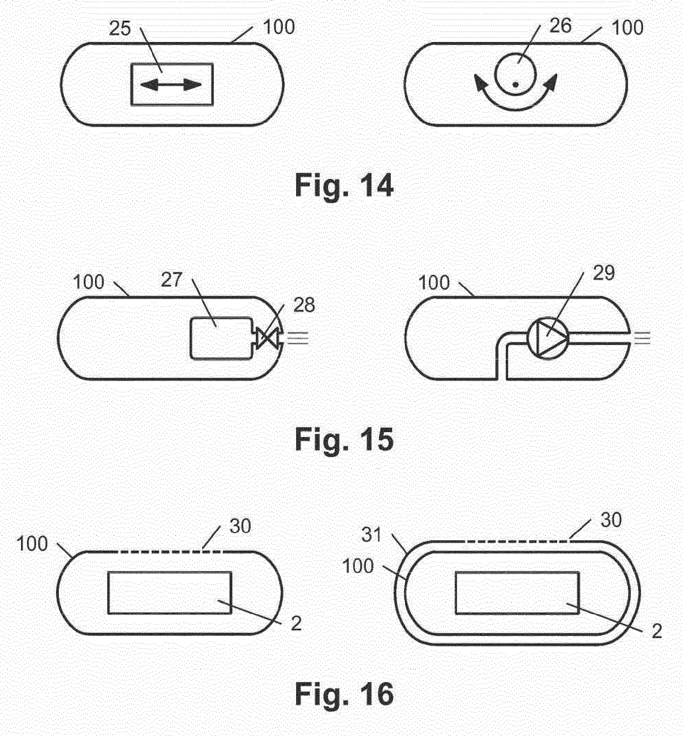

Weil das elektronische Gerät 3 (insbesondere der Mikroprozessor 5 und die Energiequelle 21) durch das Glasgehäuse 101, 102 gut vor äußeren chemischen Einflüssen geschützt ist und das Glasgehäuse 101, 102 selbst praktisch nicht mit anderen chemischen Substanzen reagiert sowie selbst keine Stoffe abgibt, eignet sich das elektronische Gerät 3 insbesondere für den Einsatz in organischem Gewebe, zum Beispiel in menschlichen und tierischen Körpern sowie in pflanzlichem Gewebe. Vorteilhaft ist dabei auch, dass das Gehäuse 101, 102 keine scharfen Kanten aufweist. Beispielsweisen kann die Analyse- und Auswerteeinheit 22 zur Analyse und Auswertung von Körperflüssigkeiten und/oder Gewebeproben ausgebildet sein. Möglich ist auch, das elektronische Gerät 3 mit Mitteln zur Fortbewegung auszustatten. In den Figuren 14 und 15 sind dazu einige beispielhafte Prinzipien gezeigt.

Die Energiequelle 21 kann auch mit einem berührungslos beaufschlagbaren Energieumsetzer verbunden sein, sodass Energie berührungslos und vor allem durch die durchgehende Gehäusewand 101, 102 hindurch übertragen werden kann. Beispielsweise kann der Energieumsetzer induktiv wirken oder zum Beispiel auch Bewegungsenergie der Kapsel in elektrische Energie umsetzen, so wie dies etwa bei Automatikuhren durchgeführt wird.

Von Vorteil ist es, wenn zumindest einer der nachstehenden Bauteile im Gehäuse 101, 102, wie Lichtquelle 13, Bilderkennungsvorrichtung 4, Bildaufnahmevorrichtung, elektronisches Gerät, Analyse-, Speicher- und Auswerteeinheit 22 mit einem Sender 5, 6 zum drahtlosen Übermitteln von Daten verbunden ist. Dadurch die Daten an externe Verarbeitungseinheiten (z.B. abgesetzter PC) übertragen werden können. Von Vorteil ist es auch, wenn zumindest einer der nachstehenden Bauteile im Gehäuse 100, wie Lichtquelle 13, Bilderkennungsvorrichtung 4, Bildaufnahmevorrichtung, elektronisches Gerät, Analyse- und Auswerteeinheit 22 mit einer Energiequelle 21 verbunden ist, da diese dann unabhängig von einer externen Energieversorgung betrieben werden können.

Die Figuren 12 und 13 zeigen nun zwei weitere Möglichkeiten zur Herstellung eines Gehäuses. In der Fig. 12 wird beispielsweise eine kegelförmige Stirnwand 103 auf ein Gehäuseteil 104 aufgesetzt und verschweißt/verklebt, und in der Fig. 13 ist ein Gehäuse gezeigt, bei dem die Gehäuseteile 105 und 106 einen Überlappungsbereich aufweisen, an dem sie beispielsweise verschweißt oder verklebt werden können.

Wie erwähnt zeigen die Figuren 14 und 15 nun Fortbewegungsmittel für die elektronischen Geräte 3. Im linken Bild der Fig. 14 ist eine Masse 25 innerhalb des Gehäuses 100 angeordnet, welche translatorisch ausgelenkt werden kann, beispielsweise mit Hilfe eines Elektromagneten. Wird die Masse 25 beispielsweise nach links langsam bewegt, nach rechts dagegen schnell, so wird die Kapsel in ihrer Gesamtheit nach links bewegt. Durch wiederholtes Auslenken kann die Kapsel nun fortbewegt werden.

Das rechte Bild der Fig. 14 zeigt ein ganz ähnliches Prinzip, allerdings mit einer exzentrisch und drehbar gelagerten Masse 26. Diese kann wie die Masse 25 hin und her bewegt werden oder aber auch stets in eine Richtung, insbesondere mit variierender Winkelgeschwindigkeit. Dadurch kann die Kapsel nun wiederum fortbewegt werden. Wegen der drehbar gelagerten Masse sind einerseits lineare Bewegungen in alle Richtungen quer zur Drehachse (Masse 26 wird hin und her bewegt) sowie Drehungen um diese Drehachse (Masse 26 wird in eine Richtung gedreht) möglich. Wird die Masse 26 um zwei quer aufeinander stehende Achsen drehbar gelagert oder werden zwei Massen 26 vorgesehen, welche um verschiedene Achsen drehbar gelagert sind, so kann die Kapsel in jede beliebige Richtung bewegt oder gedreht werden.

Die Figur 15 zeigt darüber hinaus Antriebe nach dem Rückstoßprinzip. Im linken Bild ist ein unter Druck stehender Behälter 27 vorgesehen, aus welchem der Inhalt mit Hilfe des Ventils 28 kontrolliert abgelassen und die Kapsel auf diese Weise entsprechend fortbewegt werden kann. Im Inneren des Behälters 27 können auch Substanzen chemisch miteinander reagieren, die eine Druckerhöhung im Behälter 27 bewirken.

Das rechte Bild zeigt einen Rückstoßantrieb (Jetantrieb) mit Hilfe einer Pumpe 29 für flüssige Medien respektive mit Hilfe eines Verdichters für gasförmige Medien, je nachdem in welchem Medium die Kapsel eingesetzt wird.

Selbstverständlich können die in den Figuren 14 und 15 gezeigten Prinzipien auch beliebig kombiniert werden. Beispielsweise kann die Pumpe/der Verdichter 29 mit einer drehbar gelagerten Masse 26 kombiniert werden, sodass die Kapsel in jede beliebige Richtung bewegt werden kann. Zur Drehung der Kapsel können natürlich auch radial nach außen weisende Lenkdüsen beziehungsweise Lenköffnungen verwendet werden.

Die linke Abbildung der Fig. 16 zeigt nun ein elektronisches Gerät 3 in einem Gehäuse 100, in dem Mikrobohrungen 30 angeordnet sind, die gasdurchlässig jedoch flüssigkeitsundurchlässig sind. Mit Hilfe dieser Mikrobohrungen 30 kann das elektronische Gerät 3 gekühlt werden. Zudem kann auf diese Weise die Bildung eines unerwünschten Differenzdrucks zwischen dem Inneren des Gehäuses 100 und seiner Umgebung vermieden werden, beispielsweise wenn das im Inneren des Gehäuses 100 befindliche Medium erwärmt oder abgekühlt wird. Auf diese Weise kann ein Explodieren oder Implodieren des Gehäuses 100 wirksam vermieden werden, was insbesondere bei Anwendung im menschlichen oder tierischen Körper ungewollte Konsequenzen hätte.

Es kann jedoch unerwünscht sein, dass das elektronische Gerät 3 Gasen ausgesetzt ist, das durch die Mikrobohrungen 30 hindurch dringt. Aus diesem Grund ist in einer weiteren bevorzugten Variante vorgesehen, dass das Gehäuse 100 mit dem elektronischen Gerät 3 in ein weiteres hermetisches Glasgehäuse 31 eingebracht wird, in dem Mikrobohrungen 30 angeordnet werden, die gasdurchlässig jedoch flüssigkeitsundurchlässig sind. Das innere Gehäuse 2 braucht in diesem Fall keine Mikrobohrungen aufweisen. Auf diese Weise kann das elektronische Gerät 3 gekühlt werden, ohne das es den durch die Mikrobohrungen 30 hindurch tretenden Gasen ausgesetzt ist. Wird das Gehäuse 100 dennoch durch Implosion oder Explosion zerstört, so wird das zertrümmerte Gehäuse 100 im Inneren des Gehäuses 31 gehalten und kann keinen weiteren Schaden anrichten.

Generell ist es von Vorteil, wenn eine Wandstärke des Gehäuses 100 und/oder 31, zumindest im Mittelbereich zwischen zwei Stirnwänden zwischen 0,05 bis 5 mm beträgt, um eine hinreichende mechanische Stabilität zu gewährleisten.

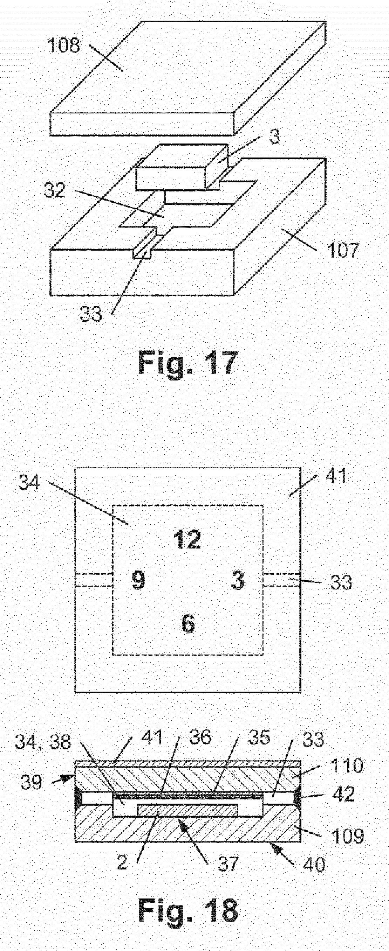

Fig. 17 zeigt nun eine Zwischenstufe bei der Herstellung eines Gehäuses für ein elektronisches Gerät nach einem anderen Verfahren. Konkret zeigt Fig. 17 ein beispielhaftes erstes Gehäuseteil 107 und ein beispielhaftes zweites Gehäuseteil 108 aus Glas für das genannte Gehäuse sowie ein elektronisches Gerät 3 in Explosionsdarstellung. Das Verfahren zur Herstellung des Gehäuses 107, 108 wird nun anhand der Fig. 17 näher erläutert.

Das Verfahren umfasst die Schritte:
- Anfertigen zumindest einer Vertiefung 32 in zumindest einem Gehäuseteil 107 eines Gehäuses,
- Herstellen zumindest eines Hohlraums durch Zusammenfügen der Gehäuseteile 107, 108, wobei zumindest eine Öffnung 33, insbesondere zumindest zwei Öffnungen 33, von aussen in den Hohlraum offen bleibt/bleiben,
- Einbringen eines elektronischen Gerätes 3 in den zumindest einen Hohlraum durch die zumindest eine Öffnung 33 und
- Verschließen und Verschweißen der zumindest einen Öffnung 33 mittels Laserstrahlung.
Insbesondere können die beiden Gehäuseteile 107, 108 mittels Laserstrahlung mit Energieabgabe im Nano- und/oder Piko-Sekundenbereich und/oder bei kontinuierlicher Energieeinbringung miteinander verschweißt werden.

Beispielsweise kann die Vertiefung 32 in das Gehäuseteil 107 gefräst werden. Denkbar ist auch, dass die Vertiefung 32 mit Hilfe eines Ionenstrahls, einem Abtragverfahren z.B. mit einem Laser oder Powder-Blasting hergestellt wird. Desgleichen können auch die für die Zuleitungsöffnungen 33 vorgesehenen Nuten beispielsweise gefräst oder mit Hilfe eines Ionenstrahls hergestellt werden. Durch Aufsetzen des zweiten Gehäuseteils 108 auf das erste Gehäuseteil 107 entsteht dann ein Hohlraum mit zwei Zuleitungsöffnungen 33. Beispielsweise kann das Gehäuseteil 108 auf das Gehäuseteil 107 aufgeklebt oder mit diesem verschweißt werden, insbesondere mit Hilfe eines Lasers.

Auf wenigstens einer Begrenzungswand des Hohlraums kann eine fluoreszierenden und/oder phosphoreszierenden Schicht hergestellt werden. Das Verfahren umfasst dann die Schritte:
- Anfertigen zumindest einer Vertiefung 32 in das erste Gehäuseteil 107,
- Herstellen zumindest eines Hohlraums durch Abdecken der zumindest einen Vertiefung 32 mit dem zumindest diffusen zweiten Gehäuseteil 108 wobei zwei Zuleitungsöffnungen 33, von aussen in den Hohlraum offen bleibt/bleiben,
- Herstellen einer fluoreszierenden und/oder phosphoreszierenden Schicht aus einem durch Zerfallsstrahlung zum Leuchten anregbaren Stoff auf wenigstens einer Begrenzungswand des zumindest einen Hohlraums,
- Einbringen eines eine Zerfallsstrahlung für einen zum Leuchten anregbaren Stoff abgebendes Medium in den zumindest einen Hohlraum durch die zumindest eine Zuleitungsöffnung und
- Verschmelzen/Verschweißen der zumindest einen Öffnung nach dem Einbringen des elektronischen Geräts 3.

Für ein Verfahren zur Herstellung selbstleuchtender Körper kann das Verfahren auch folgende Schritte umfassen:
- Anfertigen zumindest einer Vertiefung 32 in zumindest einem Gehäuseteil 107 eines Gehäuses,
- Herstellen einer fluoreszierenden und/oder phosphoreszierenden aus einem durch Zerfallsstrahlung zum Leuchten anregbaren Stoff gebildeten Schicht 36 und/oder einer Maske 41 auf wenigstens einem Teil einer Begrenzungswand 37 zumindest eines Hohlraums 34,
- Herstellen des zumindest eines Hohlraums 34 durch Zusammenfügen der Gehäuseteile 107, 108
- Verschweißen der Gehäuseteile 107, 108 mittels einer Laserstrahlung durch Abgabe von Nano- und/oder Piko-Sekunden Energieimpulsen und/oder bei kontinuierlicher Energieeinbringung, wobei zumindest eine Zuleitungsöffnung 33, insbesondere zumindest zwei Zuleitungsöffnungen 33, von aussen in den Hohlraum 34 offen bleibt/bleiben,
- Einbringen eines eine Zerfallsstrahlung für einen zum Leuchten anregbaren Stoff 36 abgebendes Medium 38 oder des Stoffs 36 und des Mediums 38 in den zumindest einen Hohlraum 34 durch die zumindest eine Zuleitungsöffnung 33 und
- Verschließen und Verschweißen der zumindest einen Zuleitungsöffnung 33 mittels Laserstrahlung.

Beispielsweise kann die fluoreszierenden und/oder phosphoreszierenden Schicht dadurch hergestellt werden, dass das wenigstens ein Gehäuseteil 107, 108 mit Klebstoff (z.B. Phosphorsäure H₃PO₄) beschichtet wird und anschließend ein fluoreszierender und/oder phosphoreszierender Stoff (z.B. Zinksulfid ZnS) auf die Klebeschicht aufgebracht wird.

Denkbar ist, dass der Klebstoff und im Anschluss der fluoreszierende und/oder phosphoreszierende Stoff, welcher die fluoreszierende und/oder phosphoreszierende Schicht bildet, durch eine der beiden Zuleitungsöffnungen 33 in den Hohlraum eingebracht wird. Dazu kann eine der beiden Zuleitungsöffnungen 33 mit einer Zuflussleitung und die andere Zuleitungsöffnung 33 mit einer Abflussleitung verbunden werden. Klebstoff kann in Form einer Flüssigkeit oder in Form eines Nebels über die Zuflussleitung in den Hohlraum eingebracht und überflüssiger Klebstoff über die Abflussleitung abgeführt werden. Auf dieselbe Weise kann auch der fluoreszierende und/oder phosphoreszierende Stoff in den Hohlraum eingebracht beziehungsweise aus diesem abgeführt werden, entweder über dieselben Leitungen oder über gesonderte Leitungen.

In einer weiteren Variante des Verfahrens wird die Klebeschicht auf das Gehäuseteil 107 und/oder das Gehäuseteil 108 aufgebracht, bevor die beiden Teile zusammengefügt werden. In einem weiteren Schritt werden das Gehäuseteil 107 und das Gehäuseteil 108 zusammengefügt, und anschließend wird der fluoreszierende und/oder phosphoreszierende Stoff über die Zuleitungsöffnungen 33 eingebracht. Diese Variante hat den Vorteil, dass der Klebstoff sehr selektiv, im Speziellen unter Zuhilfenahme einer Maske auf wenigstens eines der Gehäuseteile 107, 108 aufgetragen, beispielsweise aufgesprüht oder aufgewalzt, werden kann. Denkbar ist auch, dass der Klebstoff aufgedruckt oder aufgestempelt wird und so eine selektive Benetzung des Gehäuseteils 107 und/oder des Gehäuseteils 108 mit Klebstoff hergestellt werden kann. Der selektive Klebstoffauftrag kann beispielsweise in Form von Buchstaben, Zahlen, Symbolen oder anderen geometrischen Figuren respektive beliebigen Flächen erfolgen. Wird anschließend der fluoreszierende und/oder phosphoreszierende Stoff in den Hohlraum eingebracht, so lagert er sich an den benetzen Flächen ab und bildet ebenso Buchstaben, Zahlen, Symbole, etc. Weiterhin ist denkbar, dass nicht nur der Klebstoff auf das Gehäuseteil 107 und/oder das Gehäuseteil 108, sondern auch der fluoreszierende und/oder phosphoreszierende Stoff auf die Klebstoffschicht aufgetragen wird, bevor die Gehäuseteile 107, 108 zusammengefügt werden. Möglich ist schließlich auch, dass der der fluoreszierende und/oder phosphoreszierende Stoff selbst haftende oder klebende Eigenschaften hat. Ein getrennter Klebstoffauftrag kann dann entfallen. Beispielsweise kann direkt ein Gemisch aus Phosphorsäure (H₃PO₄) und Zinksulfid (ZnS) aufgetragen werden.

In den fertig gestellten und mit der fluoreszierenden und/oder phosphoreszierenden Schicht versehenen Hohlraum wird sodann ein selbstleuchtenden beziehungsweise zum Leuchten anregbaren Mediums (z.B. Tritium-Gas) eingebracht, wobei die beiden Zuleitungsöffnungen 33 wiederum als Zufluss und Abfluss fungieren können.

In einem weiteren Schritt werden die Zuleitungsöffnungen 33 verschlossen, beispielweise verklebt oder verschweißt.

Fig. 18 zeigt nun eine Draufsicht auf und einen Querschnitt durch ein beispielhaftes Gehäuse für das elektronische Gerät 3. Dabei sind wieder ein Gehäuseteil 109 mit einem Gehäuseteil 110 verbunden, wodurch aus einer Vertiefung 32 ein Hohlraum 34 und aus Nuten im Gehäuseteil 109 Zuleitungsöffnungen 33 entstehen. In diesem Beispiel wird angenommen dass die Oberseite des Hohlraums 34 vollflächig mit einer Klebstoffschicht 35 und darauf einer fluoreszierenden und/oder phosphoreszierenden Schicht 36 ausgestattet ist. Somit ist die Oberfläche 37 des Hohlraums 34 teilweise mit der fluoreszierenden und/oder phosphoreszierenden Schicht 36 ausgestattet. Durch ein im Hohlraum 34 befindliches Medium 38, welches eine Zerfallsstrahlung abgibt, beginnt die Schicht 36 zu leuchten.

Auf der dem Hohlraum 34 abgewandten äußeren Oberfläche 39 des Gehäuseteils 110 selbst (also auf der der Grundfläche 40 gegenüberliegenden Seite) ist noch eine lichtundurchlässige oder wenigstens lichtabschwächende Maske 41 angeordnet. In dieser Schicht sind Löcher in Form der Zahlen 3, 6, 9 und 12 vorgesehen. Wie leicht vorstellbar ist, dringt das im Hohlraum 34 respektive in der fluoreszierenden und/oder phosphoreszierenden Schicht 36 erzeugte Licht durch diese Löcher, wodurch ein selbstleuchtendes Ziffernblatt einer Uhr gebildet werden kann. Die Zahlen erscheinen dabei hell auf dunklem Grund.

Selbstverständlich wäre es auch möglich, die Maske 41 wegzulassen und stattdessen die Zahlen direkt mit Hilfe des fluoreszierenden und/oder phosphoreszierenden Stoffes 36 zu bilden, wozu eines der zuvor genannten Verfahren angewendet werden kann. Die Zahlen erscheinen dann ebenfalls hell auf dunklem Grund. Denkbar wäre auch, die Zahlen im Negativ abzubilden. Die Zahlen erscheinen dann dunkel auf hellem Grund. Insbesondere wenn keine Maske 41 verwendet wird, kann die vorgestellte Anordnung auch direkt als Uhrglas oder überhaupt als Uhrkörper verwendet werden. Beispielsweise könnten sich die Zeiger in dem Hohlraum 34 dieser Anordnung bewegen. Das Gehäuseteil 110 könnte aber auch als LCD-Display ausgestaltet sein, wodurch sich eine hintergrundbeleuchtete Anzeige realisieren lässt. Selbstverständlich kann die Maske 41 auch zwischen dem Gehäuseteil 109 und der Gehäuseteil 110 angeordnet sein. Das elektronische Gerät 3 selbst kann in diesem Anwendungsfall einen Schaltkreis zur Bestimmung der Uhrzeit und beispielsweise auch die Motoren zum Antrieb von Uhrzeigern (nicht dargestellt) umfassen.

Generell können das Gehäuseteil 109 und das Gehäuseteil 110 sowie gegebenenfalls die Maske 41 mittels Fusion Bonding (Bindung der Grenzflächen durch Van der Waal'sche Kräfte) oder auch mittels Anodischem Bonding (chemische Bindung an den Grenzflächen, welche durch elektrische Anziehungskräfte eingeleitet wird) miteinander verbunden werden. Die Gehäuseteile 109 und 110 sowie die Maske 41 können aber auch mit Hilfe eines Lasers zusammengeschweißt werden. Die Zuleitungsöffnungen 33 können wie in Fig. 18 dargestellt mit Hilfe eines Lasers (z.B. CO₂-Laser, Faserlaser, etc.) verschweißt oder aber auch verklebt oder mit einem Stopfen versehen werden.

In den Figuren 17 und 18 wurden Gehäuse 107..110 mit nur einem Hohlraum 34 dargestellt. Selbstverständlich kann ein Gehäuse 107..110 auch mehr als einen Hohlraum 34 umfassen. Diese können beispielsweise kettenartig mit Verbindungsleitungen verbunden sein und/oder jeweils mit nach außen führenden Zuleitungsöffnungen 33 versehen sein.

Weiterhin ist es möglich, dass ein Hohlraum 34 über nur eine Zuleitungsöffnung 33 oder aber auch über drei und mehr Zuleitungsöffnungen 33 verfügt. Insbesondere wenn nur eine Zuleitungsöffnung 33 zu einem Hohlraum 34 führt, können beispielsweise konzentrische Leitungen für Zu- und Abfluss des in den / aus dem Hohlraum zu befördernden Stoffes dienen.

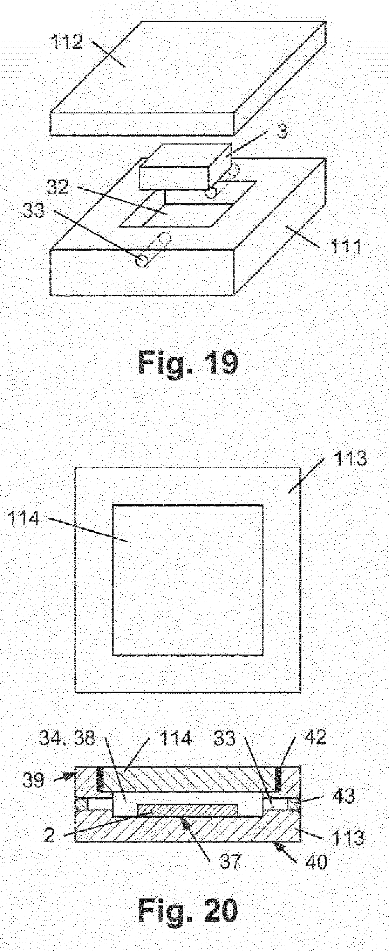

Fig. 19 zeigt eine weitere Variante eines Gehäuses 111.. 112 für ein elektronisches Gerät 3, welche der in Fig. 17 dargestellten Variante sehr ähnlich ist. Anstelle einer Nut sind hier aber Bohrungen als Zuleitungsöffnungen 33 (z.B. im Bereich von 3 µm bis 2 mm Durchmesser) vorgesehen. Diese können beispielsweise mechanisch mit Hilfe eines Bohrers, mit einem Laserstrahl oder Ionenstrahl hergestellt werden.

Fig. 20 zeigt eine weitere Variante eines Gehäuses für ein elektronisches Gerät 3, bei dem im Gehäuseteil 113 wie in Fig. 19 Löcher als Zuleitungsöffnungen 33 angeordnet sind. Im Gegensatz zu der Variante aus Fig. 16 ist das Gehäuseteil 114 hier aber etwas kleiner als das Gehäuseteil 113 und wird in eine Vertiefung desselben eingesetzt. Das Gehäuseteil 113 und das Gehäuseteil 114 sind in diesem Fall mit Hilfe einer Schweißnaht 42 miteinander verschweißt. Weiterhin ist die Zuleitungsöffnung 114 nicht wie in Fig. 16 verschweißt, sondern mit einem Stopfen 43 verschlossen. Der selbstleuchtende Körper ist in diesem Beispiel nicht als Ziffernblatt oder Uhrglas, sondern als Beleuchtungskörper ausgeführt. Das elektronische Gerät 3 kann in diesem Fall beispielsweise als berührungslos auslesbarer Transponder (RFID-Tag) ausgeführt sein, welcher eine Identifikation des Beleuchtungskörpers enthält.

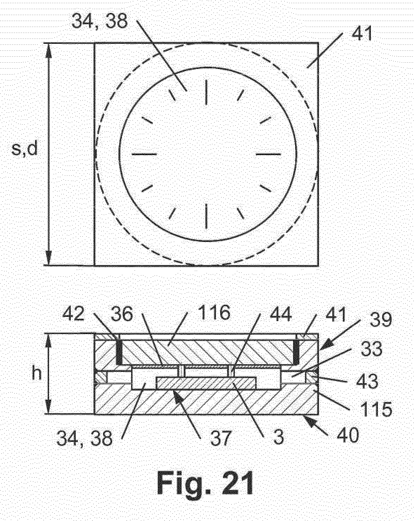

Fig. 21 zeigt nun ein Gehäuse 115, 116 welches dem in Fig. 20 dargestellten Gehäuse 113, 114 sehr ähnlich ist. Im Unterschied dazu ist aber auf dem Gehäuseteil 116 eine rahmenförmige Maske 41 aufgesetzt, welche das Durchscheinen von Licht im Randbereich des selbstleuchtenden Körpers verhindert.

In der Fig. 21 ist die fluoreszierende und/oder phosphoreszierende Schicht 36 beispielhaft an der Oberseite des Hohlraums 34 angeordnet, und zwar direkt an dem Gehäuseteil 116. Diese ist derart strukturiert, dass sich ein Ziffernblatt einer Uhr ergibt. Eine gesonderte Klebeschicht ist nicht vorgesehen, beispielsweise weil direkt ein Gemisch aus Phosphorsäure (H₃PO₄) und Zinksulfid (ZnS) und/oder Zinkoxid (ZnO) aufgetragen wurde.

Des Weiteren sind im Hohlraum 34 in der Fig. 21 Stützen 44 vorgesehen, sodass sich die Gehäuseteile 115, 116 nicht übermäßig durchbiegen können. Beispielsweise können die Stützen 44 direkt an das Gehäuseteil 115 oder Gehäuseteil 116 angeformt und mit dem jeweils anderen Gehäuseteil 115, 116 beispielsweise verklebt sein. Denkbar ist natürlich auch, dass die Stützen 44 das jeweils andere Gehäuseteil 115, 116 lediglich berühren, das heißt nicht mit diesem dauerhaft verbunden sind. Als weitere Möglichkeit können die Stützen 44 auch als gesonderte Bauteile vorliegen, die mit einem Gehäuseteil 115, 116 oder beiden Gehäuseteilen 115, 116 verbunden werden. Schließlich können die Stützen 44 auch wie in Fig. 21 dargestellt das elektronische Gerät 3 berühren oder mit diesem verbunden sein.

Generell können die in den Figuren 27 bis 21 dargestellten selbstleuchtenden Körper als Quader ausgebildet sein und somit eine rechteckige oder quadratische Grundfläche 40 aufweisen. Denkbar sind natürlich auch andere Formen. Insbesondere kann die Grundfläche 40 elliptisch oder kreisförmig ausgebildet sein (siehe dazu den alternativen strichliert dargestellten Umriss in der Draufsicht der Fig. 21). Im Speziellen kann der selbstleuchtender Körper ein als Quader oder Scheibe ausgebildetes Gehäuse 107..116 aufweisen, das durch zwei im Wesentlichen plattenförmige Gehäuseteile 107..116 mit einer mehreckigen oder elliptischen oder kreisrunden Grundfläche 40 gebildet wird, wobei die Summe der zur Grundfläche senkrechten Höhen h der beiden Gehäuseteile 107..116 geringer ist als eine kürzere Seitenlänge s bzw. ein minimaler Durchmesser d oder Radius derselben.

Generell kann das elektronische Gerät 3 der Figuren 17 bis 21 eine Kamera 4 umfassen. In diesem Fall kann die fluoreszierenden und/oder phosphoreszierenden Schicht 36 dazu dienen, das Sichtfeld der Kamera 4 auszuleuchten und ist dann nicht notwendigerweise in Form von Buchstaben und dergleichen ausgeführt. Umgekehrt kann die in Bezug auf die Figuren 17 bis 21 offenbarte Lehre im Hinblick auf die Aufbringung einer fluoreszierenden und/oder phosphoreszierenden Schicht auch auf die Ausführungsformen der Figuren 1 bis 17 angewandt werden.

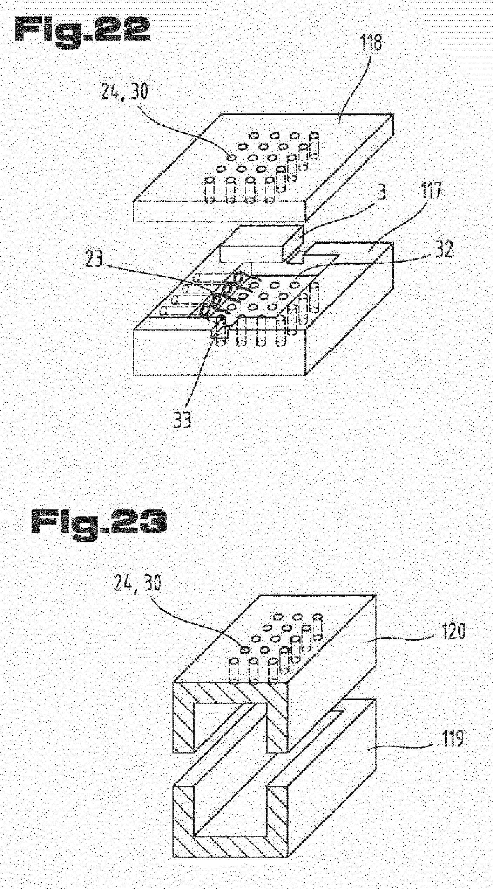

Fig. 22 zeigt nun eine weitere Ausführungsform, welche die Merkmale der Fig. 12 bzw. Fig. 16 und der Figuren 17 bis 21 kombiniert. Konkret sind in den Gehäuseteilen 117, 118 Durchtrittslöcher 24 respektive Mikrobohrungen 30 angeordnet. Des Weiteren sind elektrische Leiter 23 im Gehäuseteil 117 angeordnet, welche der Konktaktierung des elektronischen Geräts 3 dienen.

Fig. 23 zeigt eine alternative Form von Gehäuseteilen 119, 120 welche im Wesentlichen U-förmig ausgestaltet sind. Durch Verbinden der Gehäuseteile 119, 120 entsteht ein einseitig geöffneter Hohlkörper, der beispielsweise durch eine nicht dargestellte Stirnwand verschlossen werden kann.

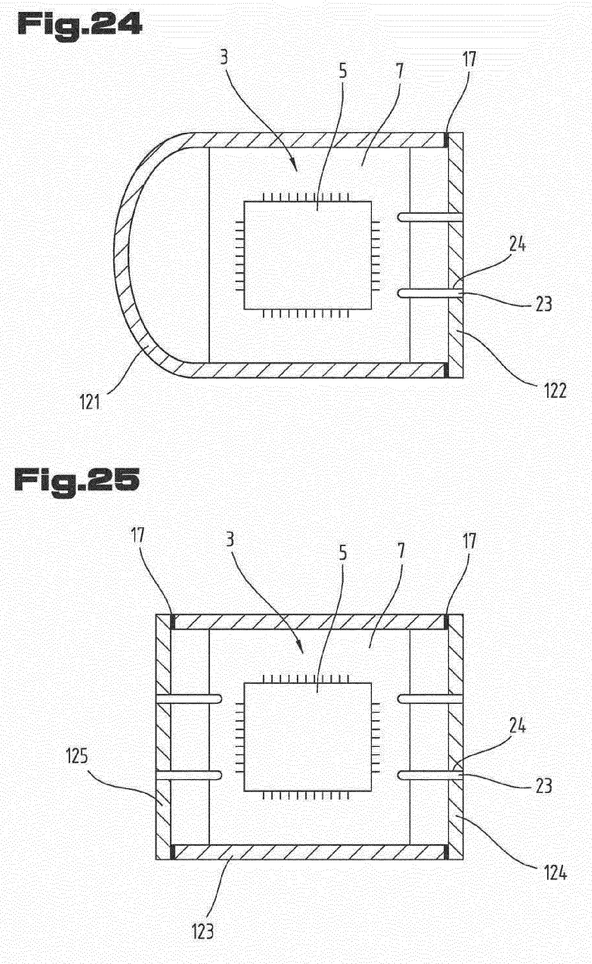

Fig. 24 zeigt ein weiteres beispielhaftes und schematisch dargestelltes Gehäuse mit einem darin angeordneten elektronischen Gerät 3. Der Gehäuseteil 121, welcher ähnlich geformt ist wie eine Eprouvette, ist mit Hilfe einer Schweißnaht 17 mit einem plattenförmigen Gehäuseteil 121 verbunden. Die beiden Gehäuseteile 121 und 122 bestehen in diesem Beispiel aus Glas und können insbesondere mit einem Laser verschweißt sein. Das schematisch dargestellte elektronische Gerät 3 umfasst in diesem Beispiel einen Mikroprozessor 5, der auf eine Leiterplatte 7 aufgelötet ist. Weiterhin sind mit der Leiterplatte 7 elektrische Leiter 23 verbunden, welche durch Durchtrittslöcher 24 geführt sind, und beispielsweise der elektrischen Stromversorgung, als Steuerleitungen oder dem Abgriff von Sensorsignalen dienen können.

Fig. 25 zeigt schließlich eine Anordnung, welche der in Fig. 24 dargestellten Anordnung sehr ähnlich ist. Im Unterschied dazu umfasst das Gehäuse allerdings einen rohrförmigen Gehäuseteil 123, der beidseits mit plattenförmigen Gehäuseteilen 124 und 125 verbunden, insbesondere wieder verschweißt, ist.

Die in den Figuren dargestellten Varianten des Gehäuses 100..125 für ein elektronisches Gerät 3 zeigen gegebenenfalls für sich eigenständige Ausführungsformen, wobei für gleiche Teile gleiche Bezugszeichen bzw. Bauteilbezeichnungen verwendet werden.

Die zu den unterschiedlichen Varianten gezeigten speziellen Ausführungsdetails beziehen sich nicht zwangsläufig nur auf die betreffende Figur, sondern können gegebenenfalls auch in anderen Ausführungsformen angewandt werden. Beispielsweise können die Isolationsschichten der Fig. 9 sinngemäß auch in einer Variante nach den Figuren 17 bis 23 angewandt werden. Gleichermaßen ist vorstellbar, dass die Variante nach den Figuren 17 bis 23 so wie in Fig. 10 gekühlt werden.

Weiterhin wird darauf hingewiesen, dass die Anwendung der präsentierten Anordnungen natürlich nicht auf den Uhrenbau beschränkt ist. Denkbar ist beispielsweise auch die Anwendung als Hinweisschild, Notbeleuchtung, Türschild, Tastaturhintergrundbeleuchtung und ähnliches.

Wie erwähnt können die vom Glasgehäuse 100..125 umschlossenen elektronischen Geräte 3 insbesondere auch im menschlichen und tierischen Körper sowie in Pflanzen eingesetzt werden. Dabei ist es möglich, dass die Geräte 3 operativ implantiert werden. Zu diesem Zweck kann die äußere Oberfläche des Gehäuses 100.. 125 aufgeraut und/oder mit reaktiven, ein Anwachsen in menschlichen/tierischen/pflanzlichen Gewebe begünstigen Substanzen/Strukturen versehen sein. Es ist aber auch möglich andere Beschichtungen, Überzüge oder Lagen wie Silikone, antistatische, bakterienhemmende, schmutzabweisende und/oder anhaftende Materialien aufzubringen.

Das Gehäuse 100..125 bzw. ein hermetisch abgeschlossener Teilraum 19, 20 desselben ist diffusionsdicht d.h. die Wasserdampfdurchlässigkeit (s_{d}) ist bevorzugt größer als 2.500m basierend auf den Festlegungen in der DIN 4108-3.

Insbesondere die in den Figuren 1 bis 16 dargestellten rundlichen Kapseln können auch zum Verschlucken vorgesehen sein. Vorteilhaft kann die äußere Oberfläche des Gehäuses mit einem Gel und/oder einem Geschmacksträger beschichtet sein, um das Verschlucken zu erleichtern.

Selbstverständlich kann aber auch eine Ausführungsform nach den Figuren 17 bis 23 zum Verschlucken und eine Ausführungsform nach den Figuren 1 bis 16 zum Implantieren vorgesehen sein. Vorteilhaft ist die Oberfläche des Gehäuses dabei zum jeweiligen Zweck behandelt.

Generell können Gehäuseteile 101..125 völlig transparent, diffus oder opak gestaltet sein (Zwischenstufen sind dabei natürlich möglich). Beispielsweise können die diffusen oder opaken Teile des Gehäuses 100..125 bzw. der Deckschicht den Schmelz- bzw. Schweißbereichen 8 benachbart angeordnet sein, wodurch es insbesondere möglich ist, Gehäuseteile 101..125 mit unterschiedlichen optischen Eigenschaften miteinander zu verbinden. Eine weitere Möglichkeit ist es auch, das Gehäuse 100..125 oder zumindest einen Teil des Gehäuses 100..125 bzw. der Deckschicht mit einer Funktionsbeschichtung, z.B. einer Folie, zu versehen, die verschiedene optische Eigenschaften aufweist. Beispielsweise kann die Funktionsbeschichtung, z.B. die Folie, diffus oder opak ausgebildet sein. Denkbar ist natürlich auch die Beeinflussung anderer physikalischer Eigenschaften mit Hilfe einer Funktionsbeschichtung, wie zum Beispiel der elektrischen Leitfähigkeit.

Der Ordnung halber sei abschließend darauf hingewiesen, dass zum besseren Verständnis des Aufbaus der vorgestellten Anordnungen diese beziehungsweise deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

Vor allem können die einzelnen in den Figuren 1 bis 23 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 100...125: Gehäuse, Gehäuseteile
- 2: rohrförmiger Grundkörper
- 3: elektronisches Gerät
- 4: Kamera
- 5: Mikroprozessor

- 6: Antenne
- 7: Leiterplatte
- 8: Schmelz-/Schweißbereich/Trennbereich
- 9: Laser
- 10: Stirnwand

- 11: (rohrförmiger) Mittelteil
- 12: Hohlraum
- 13: Tritiumgaslichtquelle
- 14: thermische Barriere / Isolation
- 15: Kühlmanschette

- 16: Spülbohrung
- 17: Schweißnaht
- 18: Trennwand
- 19: erster Hohlraum
- 20: zweiter Hohlraum

- 21: Energiequelle
- 22: Meßwertaufnehmer
- 23: elektrischer Leiter
- 24: Durchtrittslöcher
- 25: linear bewegliche Masse

- 26: exzentrisch gelagerte drehbare Masse
- 27: Druckbehälter
- 28: Ventil
- 29: Pumpe / Verdichter
- 30: Mikrobohrung

- 31: Außengehäuse
- 32: Vertiefung
- 33: Zuleitungsöffnung
- 34: Hohlraum
- 35: Klebstoffschicht
- 36: Fluoreszierende und/oder phosphoreszierende Schicht/Stoff
- 37: Oberfläche des Hohlraums
- 38: Zerfallsstrahlung abgebendes Medium
- 39: Oberfläche des Gehäuses
- 40: Grundfläche des Gehäuses

- 41: Maske
- 42: Schweißnaht
- 43: Stopfen
- 44: Stützen

- d: Durchmesser
- h: Höhe
- s: Seitenlänge

## Patentansprüche

1. Verfahren zur Herstellung eines Gehäuses (100..125) mit zumindest einem, zumindest einen Teil eines Innenraumes des Gehäuses (100..125) umfassenden hermetisch abgeschlossenen Aufnahmeraum (12, 19, 20) für ein elektronisches Gerät (3), umfassend die Schritte:
- Herstellen/Bereitstellen eines mindestens eine Öffnung aufweisenden Hohlkörpers (2) aus Glas,
- Einbringen, Positionieren und/oder Fixieren zumindest eines elektronischen Geräts (3) durch die zumindest eine Öffnung
- hermetisches Verschließen des Aufnahmeraums (12, 19, 20) durch Verschmelzen des Gehäuses (100..125) oder
- Verschließen und Verschweißen der zumindest einen Öffnung mittels Laserstrahlung.

2. Verfahren nach Anspruch 1, **dadurch gekennzeichnet, dass** zwischen dem Gerät (3) und dem Schmelz- bzw. Schweißbereich (8) ein Wärmeisolator oder eine Wärmeschutzschicht (14) angeordnet wird.

3. Verfahren nach Anspruch 1 oder 2, **dadurch gekennzeichnet, dass** in den Innenraum eine Zwischenwand (18) eingesetzt und mit dem Gehäuse (100..125) verschweißt wird und die zwei Aufnahmeräume (19, 20) hermetisch voneinander getrennt werden.

4. Verfahren nach einem oder mehreren der vorhergehenden Ansprüche, **dadurch gekennzeichnet, dass** in einem ersten hermetisch abgeschlossenen Aufnahmeraum (12, 19, 20) ein elektronisches Gerät (3) und/oder eine Energiequelle (21) angeordnet ist.

5. Verfahren nach einem oder mehreren der vorhergehenden Ansprüche, **dadurch gekennzeichnet, dass** in einem dem ersten hermetisch abgeschlossenen Aufnahmeraum (19) benachbarten Aufnahmeraum (20) zumindest eines der nachfolgend genannten Elemente (22), wie elektronisches Gerät, Analysevorrichtung, Messwertaufnehmer, angeordnet wird.

6. Verfahren nach einem oder mehreren der vorhergehenden Ansprüche, **dadurch gekennzeichnet, dass** in der Zwischenwand (18) zwischen dem ersten hermetischen Aufnahmeraum (19) und dem zweiten hermetischen Aufnahmeraum (20) Öffnungen (23) angeordnet sind, die durch Lichtleiter, elektrische Leitungen oder leitende Kontaktmassen hermetisch verschlossen werden.

7. Verfahren nach einem der Ansprüche 1 bis 6, **dadurch gekennzeichnet, dass** eine Kamera (4) in den Hohlraum (12, 19, 20) eingebracht wird.

8. Verfahren nach einem oder mehreren der vorhergehenden Ansprüche, **dadurch gekennzeichnet, dass** ein Funkmodul und/oder ein Transponder in den Hohlraum (12, 19, 20) eingebracht wird.

9. Verfahren nach einem der Ansprüche 1 bis 8, **dadurch gekennzeichnet, dass** eine motorisch bewegte Masse (25, 26) in den Hohlraum (12, 19, 20) eingebracht wird.

10. Verfahren nach einem der Ansprüche 1 bis 9, **dadurch gekennzeichnet, dass** ein Rückstoßantrieb (27..29) mit einer Durchtrittsöffnung in dem Gehäuse (101..106) in den Hohlraum (12, 19, 20) eingebracht wird.

11. Verfahren nach einem der Ansprüche 1 bis 10, **dadurch gekennzeichnet, dass** im Hohlraum (12, 19, 20) eine Tritiumgaslichtquelle (13) angeordnet wird.

12. Verfahren nach einem oder mehreren der vorhergehenden Ansprüche, **dadurch gekennzeichnet, dass** in ein rohrförmiges Gehäuse (100..125) durch die zumindest eine Öffnung mehrere elektronische Geräte (3) mit Abstand in Längsrichtung hintereinander eingebracht, positioniert und/oder fixiert werden, worauf das Gehäuse (100.. 125) mittels eines Laserstrahlung durch Abgabe von Nano- und/oder Piko-Sekunden Energieimpulsen und/oder bei kontinuierlicher Energieeinbringung im Zwischenbereich (8) zwischen den elektronischen Geräten (3) erwärmt und durch auf die Innen- und Außenseite des Gehäuses (100..125) im Bereich der Trennstelle (8) in Längsrichtung des Gehäuses (100..125) ausgeübte unterschiedlich große Kräfte beidseits des Zwischenbereiches das rohrförmige Gehäuse (100..125) mit einer Stirnwand (10) verschlossen wird.

13. Verfahren nach einem der Ansprüche 1 bis 12, **dadurch gekennzeichnet, dass** zur Herstellung des Gehäuses (100..125) wenigstens ein Ende eines rohrförmigen Grundkörpers (2) verschmolzen/verschweißt wird.

14. Verfahren nach einem oder mehreren der vorhergehenden Ansprüche, **dadurch gekennzeichnet, dass** eine Stirnwand (10) des Gehäuses (100..125) durch Erhitzen des Endbereiches des Mittelteils (11) mittels eines Lasers durch Abgabe von Nano- und/oder Piko-Sekunden Energieimpulsen erwärmt und durch auf die Innen- und Außenseite des Gehäuses (100..125) im Bereich der Trennstelle(8) ausgeübte unterschiedlich große Kräfte in Längsrichtung des Gehäuses (100..125) eine Stirnwand (10) gebildet wird.

15. Verfahren nach einem der Ansprüche 1 bis 14, **dadurch gekennzeichnet, dass** zur Herstellung des Gehäuses (100..125) ein rohrförmiger Grundkörper (2) durch Aufschmelzen getrennt wird, wobei der geschmolzene Werkstoff die entstehenden Enden verschließt.

16. Verfahren nach Anspruch 15, **dadurch gekennzeichnet, dass** während des Trenn- und/oder Verschließvorgangs des rohrförmigen Gehäuses (100..125) auf die Innen- und Außenseite des Gehäuses (100..125) im Bereich der Trennstelle (8) unterschiedlich große Kräfte in Längsrichtung des Gehäuses (100..125) ausgeübt werden.

17. Verfahren nach einem oder mehreren der vorhergehenden Ansprüche, **dadurch gekennzeichnet, dass** während des Trenn- und/oder Verschließvorgangs des rohrförmigen Gehäuses (100..125) die Innen- und Außenseite des Gehäuses (100..125) im Bereich der Trennstelle (8) mit einer unterschiedlich großen Druckkraft beaufschlagt wird.

18. Verfahren nach einem oder mehreren der vorhergehenden Ansprüche, **dadurch gekennzeichnet, dass** das Gehäuse (100..125) als Quader ausgebildet wird.

19. Verfahren nach einem oder mehreren der vorhergehenden Ansprüche, **dadurch gekennzeichnet, dass** das Gehäuse durch zwei im wesentlichen plattenförmige Gehäuseteile (107..125) mit einer quadratischen bzw. rechteckigen Grundfläche (40) gebildet wird und die Summe der zur Grundfläche (40) senkrechten Höhe (h) der beiden Gehäuseteile (107..125) geringer ist als eine kürzere Seitenlänge (s) derselben.

20. Verfahren nach einem der Ansprüche 1 bis 19, **gekennzeichnet, durch**:
- Anfertigen zumindest einer Vertiefung (32) in zumindest einem Gehäuseteil (107..125) eines Gehäuses,
- Herstellen zumindest eines Hohlraums (34) **durch** Zusammenfügen der Gehäuseteile (107..125), wobei zumindest eine Öffnung (33), insbesondere zumindest zwei Öffnungen (33), von aussen in den Hohlraum (34) offen bleibt/bleiben,
- Einbringen eines elektronischen Gerätes (3) in den zumindest einen Hohlraum (34) **durch** die zumindest eine Öffnung (33) und
- Verschließen und Verschweißen der zumindest einen Öffnung (33) mittels Laserstrahlung.

21. Verfahren nach einem oder mehreren der vorhergehenden Ansprüche, **dadurch gekennzeichnet, dass** der eine Gehäuseteil (107..125) durch eine plattenförmige Deckschicht, gebildet wird.

22. Verfahren nach einem der Ansprüche 1 bis 21, **dadurch gekennzeichnet, dass** in dem Gehäuse (100..125) Mikrobohrungen (30) angeordnet werden, die gasdurchlässig und flüssigkeitsundurchlässig sind.

23. Verfahren nach einem der Ansprüche 1 bis 22, **dadurch gekennzeichnet, dass** das Gehäuse (100..125) mit dem elektronischen Gerät (3) in ein weiteres hermetisches Glasgehäuse (31) eingebracht wird, in dem Mikrobohrungen (30) angeordnet werden, die gasdurchlässig und flüssigkeitsundurchlässig sind.

24. Verfahren nach einem der Ansprüche 1 bis 23, **dadurch gekennzeichnet, dass** in dem Gehäuse (3) Bohrungen für den Durchtritt metallischer Drähte (23) und/oder Lichtleitfasern angeordnet werden.

25. Verfahren nach einem der Ansprüche 1 bis 24, **dadurch gekennzeichnet, dass** die äußere Oberfläche (39) des Gehäuses (100..125) mit einem Gel und/oder einem Geschmacksträger beschichtet wird.

26. Verfahren nach einem der Ansprüche 1 bis 25, **dadurch gekennzeichnet, dass** die äußere Oberfläche (39) des Gehäuses (100..125) aufgeraut und/oder mit reaktiven, ein Anwachsen in menschlichen/tierischen/pflanzlichen Gewebe begünstigen Substanzen/Strukturen versehen wird.

27. Verfahren nach einem der vorhergehenden Ansprüche, **dadurch gekennzeichnet, dass** das Gehäuse aus zwei Gehäuseteilen (101..125) besteht und die beiden Gehäuseteile (101..125) mittels Laserstrahlung mit Energieabgabe im Nano- und/oder Piko-Sekundenbereich und/oder bei kontinuierlicher Energieeinbringung miteinander verschweißt werden.

28. Verfahren nach einem der vorhergehenden Ansprüche, **dadurch gekennzeichnet, dass** die Abgabe der Energie der Laserimpulse mit einer Steuervorrichtung derart gesteuert wird, dass die Temperatur im Inneren des Gehäuses (101..125) in einem Bereich der gleich oder größer 2 mm von der Schweißnaht oder der Einwirkstelle (8) der Laserstrahlen entfernt ist unter einem Wert von 200° gehalten wird.

29. Verfahren nach einem der vorhergehenden Ansprüche, **dadurch gekennzeichnet, dass** während des Aufbringens der Wärmeenergie durch die Laserstrahlung die der Schweißnaht (17, 42) benachbarten Bereiche gekühlt werden.

30. Verfahren zur Herstellung selbstleuchtender Körper, umfassend die Schritte:
- Anfertigen zumindest einer Vertiefung (32) in zumindest einem Gehäuseteil (108..125) eines Gehäuses,
- Herstellen einer fluoreszierenden und/oder phosphoreszierenden aus einem durch Zerfallsstrahlung zum Leuchten anregbaren Stoff gebildeten Schicht (36) und/oder einer Maske (41) auf wenigstens einem Teil einer Begrenzungswand (37) zumindest eines Hohlraums (34),
- Herstellen des zumindest eines Hohlraums (34) durch Zusammenfügen der Gehäuseteile (108..125)
- Verschweißen der Gehäuseteile (108..125) mittels einer Laserstrahlung durch Abgabe von Nano- und/oder Piko-Sekunden Energieimpulsen, wobei zumindest eine Zuleitungsöffnung (33), insbesondere zumindest zwei Zuleitungsöffnungen (33), von aussen in den Hohlraum (34) offen bleibt/bleiben,
- Einbringen eines eine Zerfallsstrahlung für einen zum Leuchten anregbaren Stoff (36) abgebendes Medium (38) oder des Stoffs (36) und des Mediums (38) in den zumindest einen Hohlraum (34) durch die zumindest eine Zuleitungsöffnung (33) und
- Verschließen und Verschweißen der zumindest einen Zuleitungsöffnung (33) mittels mittels Hitzeeinwirkung durch Laserstrahlung und/oder eine Gasflamme.

31. Verfahren nach einem oder mehreren der vorhergehenden Ansprüche, **dadurch gekennzeichnet, dass** die Laserstrahlung durch Nano- und/oder Piko-Sekunden Energieimpulsen und/oder bei kontinuierlicher Energieeinbringung gebildet wird.

32. Verfahren nach einem oder mehreren der vorhergehenden Ansprüche, **dadurch gekennzeichnet, dass** zumindest ein Teil des Gehäuses (100..125) bzw. der Deckschicht diffus hergestellt wird.

33. Vorrichtung mit einem zumindest zum Teil hermetisch abgeschlossenen Gehäuse (100..125) aus Silizium, insbesondere nach einem der Verfahrensansprüche 1 bis 32, **dadurch gekennzeichnet, dass** das Gehäuse (100..125) einteilig aus einem einzigen Grundwerkstoff hergestellt ist.

34. Vorrichtung mit einem zumindest zum Teil hermetisch abgeschlossenen Gehäuse (100..125) aus Silizium, insbesondere nach einem der Verfahrensansprüche 1 bis 32, **dadurch gekennzeichnet, dass** das Gehäuse (100..125) aus mehreren Gehäuseteilen (101..125) besteht und zumindest aus einem einzigen Grundwerkstoff hergestellt ist.

35. Vorrichtung nach einem oder mehreren der vorhergehenden Ansprüche, **dadurch gekennzeichnet, dass** die Gehäuseteile (101..125) aus verschiedenen Werkstoffen gebildet sind die im Wesentlichen dieselben physikalischen und chemischen Eigenschaften aufweisen.

36. Vorrichtung nach Anspruch 35, **dadurch gekennzeichnet, dass** zumindest eine der Stirnwände (10) konkav oder ebenflächig ausgebildet ist.

37. Vorrichtung nach Anspruch 35, **dadurch gekennzeichnet, dass** zumindest eine der Stirnwände (10) konvex ausgebildet ist.

38. Vorrichtung nach Anspruch 35, **dadurch gekennzeichnet, dass** die Stirnwände (10) mit einem sich in die vom Mittelteil (11) abgewendeten Richtung erstreckenden Konus oder als Kugelkalotte bzw. Kugelkalottenabschnitt ausgebildet sind.

39. Vorrichtung nach einem oder mehreren der vorhergehenden Ansprüche, **dadurch gekennzeichnet, dass** das Gehäuse aus einem zylindrischen Mitteilteil (11) und zwei in den Stirnseiten desselben angeordneten Stirnwänden (10) gebildet ist.

40. Vorrichtung nach einem oder mehreren der vorhergehenden Ansprüche, **dadurch gekennzeichnet, dass** zwischen dem elektronischen Gerät (3) und zumindest einer der beiden Stirnwände (10) ein Wärmeisolationsvorrichtung (14) angeordnet ist.

41. Vorrichtung nach einem oder mehreren der vorhergehenden Ansprüche, **dadurch gekennzeichnet, dass** ein Innenraum des Gehäuses (100..125) durch eine Zwischenwand (18) in zwei Teilräume (19, 20) unterteilt ist.

42. Vorrichtung nach einem oder mehreren der vorhergehenden Ansprüche, **dadurch gekennzeichnet, dass** das Gehäuse (100..125) zumindest eine von Außen sich zumindest in einem der beiden Teilräume (20) erstreckende Öffnung (24) aufweist, die luft- und/oder flüssigkeitsdurchlässig ist.

43. Vorrichtung nach einem oder mehreren der vorhergehenden Ansprüche, **dadurch gekennzeichnet, dass** beide Teilräume (19, 20) hermetisch abgeschlossen sind.

44. Vorrichtung nach einem oder mehreren der vorhergehenden Ansprüche, **dadurch gekennzeichnet, dass** der Teilraum (19, 20) gasdicht, insbesondere Tritiumgasdicht, ausgebildet ist.

45. Vorrichtung nach einem oder mehreren der vorhergehenden Ansprüche, **dadurch gekennzeichnet, dass** der Teilraum (19, 20) wasserdampfdicht mit einer Wasserdampfdurchlässigkeit s_{d} größer als 2.500m ausgebildet ist.

46. Vorrichtung nach einem oder mehreren der vorhergehenden Ansprüche, **dadurch gekennzeichnet, dass** in zumindest einem der beiden Teilräume (19, 20) eine Analyse- und/oder Auswerteeinheit (22) angeordnet ist.

47. Vorrichtung nach einem oder mehreren der vorhergehenden Ansprüche, **dadurch gekennzeichnet, dass** die Analyse- und Auswerteeinheit (22) zur Analyse und Auswertung von Körperflüssigkeiten und/oder Gewebeproben ausgebildet ist.

48. Vorrichtung nach einem oder mehreren der vorhergehenden Ansprüche, **dadurch gekennzeichnet, dass** in dem Gehäuse (100..125) zumindest eine Lichtquelle, wie eine LED, eine Tritiumgaslichtquelle (13, 36) oder ein Leuchtstoff mit Nachleuchteigenschaften angeordnet ist.

49. Vorrichtung nach einem oder mehreren der vorhergehenden Ansprüche, **dadurch gekennzeichnet, dass** im Gehäuse (100..125) eine Bildaufnahmeeinrichtung (4), zum Beispiel ein Bilderkennungschip angeordnet ist.

50. Vorrichtung nach einem oder mehreren der vorhergehenden Ansprüche, **dadurch gekennzeichnet, dass** mit zumindest eines der nachstehend Bauteile im Gehäuse (100..125), wie Lichtquelle (13, 36), Bilderkennungsvorrichtung (4), Bildaufnahmevorrichtung, elektronisches Gerät, Analyse-, Speicher- und Auswerteeinheit (22) mit einem Sender (5, 6) zum drahtlosen Übermitteln von Daten verbunden ist.

51. Vorrichtung nach einem oder mehreren der vorhergehenden Ansprüche, **dadurch gekennzeichnet, dass** zumindest eines der nachstehend Bauteile im Gehäuse (100..125), wie Lichtquelle (13, 36), Bilderkennungsvorrichtung (4), Bildaufnahmevorrichtung, elektronisches Gerät, Analyse- und Auswerteeinheit (22) mit einer Energiequelle (21) verbunden ist.

52. Vorrichtung nach einem oder mehreren der vorhergehenden Ansprüche, **dadurch gekennzeichnet, dass** die Energiequelle (21) mit einem berührungslos beaufschlagbaren Energieumsetzer verbunden ist.

53. Vorrichtung nach einem oder mehreren der vorhergehenden Ansprüche, **dadurch gekennzeichnet, dass** die Stirnwände (10) durch Zufuhr von Laserenergie, insbesondere mit Nano- oder Piko-Sekundenimpulsen und/oder bei kontinuierlicher Energieeinbringung gebildet sind.

54. Vorrichtung nach einem oder mehreren der vorhergehenden Ansprüche, **dadurch gekennzeichnet, dass** der Taupunkt des Wasserdampfes in der Luft im Gehäuse zumindest in dessen hermetisch abgeschlossenen Teilraum 0 °C bevorzugt zwischen - 10°C und -30°C beträgt.

55. Vorrichtung nach einem oder mehreren der vorhergehenden Ansprüche, **dadurch gekennzeichnet, dass** eine Wandstärke des Gehäuses (100..125) zumindest im Mittelbereich zwischen 0,05 bis 5 mm beträgt.

56. Vorrichtung nach einem oder mehreren der vorhergehenden Ansprüche, **dadurch gekennzeichnet, dass** zumindest ein Teil des Gehäuses (100..125) bzw. der Deckschicht diffus oder opak ausgebildet ist.

57. Vorrichtung nach einem oder mehreren der vorhergehenden Ansprüche, **dadurch gekennzeichnet, dass** die diffusen oder opaken Teile des Gehäuses (100..125) bzw. der Deckschicht den Schmelz- bzw. Schweißbereichen (8) benachbart angeordnet sind.

58. Vorrichtung nach einem oder mehreren der vorhergehenden Ansprüche, **dadurch gekennzeichnet, dass** zumindest ein Teil des Gehäuses (100..125) bzw. der Deckschicht mit einer Funktionsbeschichtung, z.B. einer Folie, versehen ist.

59. Vorrichtung nach einem oder mehreren der vorhergehenden Ansprüche, **dadurch gekennzeichnet, dass** die Funktionsbeschichtung, z.B. die Folie, diffus oder opak ausgebildet ist.

60. Vorrichtung nach einem oder mehreren der vorhergehenden Ansprüche, **dadurch gekennzeichnet, dass** die beiden Gehäuseteile (106..125) mit über den Hohlraum (34) verteilten, senkrecht zu den Grundflächen des Hohlraums (34) ausgerichteten Stützelementen (44) voneinander distanziert sind.

61. Vorrichtung nach einem oder mehreren der vorhergehenden Ansprüche, **dadurch gekennzeichnet, dass** die Stützelemente (44) mit zumindest einem der beiden Gehäuseteile (101..125) verbunden sind.

62. Vorrichtung nach einem oder mehreren der vorhergehenden Ansprüche, **dadurch gekennzeichnet, dass** das Gehäuse (106..125) als Quader oder Scheibe ausgebildet ist.

63. Vorrichtung nach einem oder mehreren der vorhergehenden Ansprüche, **dadurch gekennzeichnet, dass** das Quader oder die Scheibe durch zwei im Wesentlichen plattenförmige Gehäuseteile (106..125) mit einer mehreckigen oder elliptischen oder kreisrunden Grundfläche (40) gebildet wird und die Summe der zur Grundfläche (40) senkrechten Höhen (h) der beiden Gehäuseteile (106..125) geringer ist als eine kürzere Seitenlänge (s) bzw. ein minimaler Durchmesser (d) oder Radius derselben.
